# EUROPEAN PATENT SPECIFICATION

(11) **EP 2 235 045 B1**
(45) Date of publication and mention of the grant of the patent: **03.10.2012**
(21) Application number: 08870024.0
(22) Date of filing: 08.12.2008
(51) Int. Cl.: C07K 14/31, C02F 1/28, C02F 3/34, C07K 19/00

(54) **PRODUCTION OF HETEROLOGOUS PROTEINS OR PEPTIDES**
ERZEUGUNG HETEROLOGER PROTEINE ODER PEPTIDE
PRODUCTION DE PROTÉINES OU PEPTIDES HÉTÉROLOGUES

(30) Priority: 08.01.2008 ZA 200800205
(43) Date of publication of application: 06.10.2010
(73) Proprietor: CSIR, 0002 Pretoria (ZA)
(72) Inventor: BERGER, Eldie, 0081 Menlo Park (ZA); DU PLESSIS, Erika, Margarete, 0043 Pretoria (ZA); LOUW, Maureen, Elizabeth, 0181 Pretoria (ZA); CRAMPTON, Michael, Craig, 0081 Menlo Park (ZA); GERBER, Isak, Bartholomeus, 1739 Krugersdorp (ZA)
(74) Representative: Cohausz & Florack
(86) International application number: PCT/IB2008/055143
(87) International publication number: WO 2009/087508

(56) References cited:
- WO-A-2006/072845
- WO-A-2006/117582
- JP-A- 1 112 985
- US-A- 4 801 536
- MICHAEL CRAMPTON ET AL: "The development of a flagellin surface display expression system in a moderate thermophile, Bacillus halodurans Alk36" APPLIED MICROBIOLOGY AND BIOTECHNOLOGY, SPRINGER, BERLIN, DE, vol. 75, no. 3, 21 February 2007 (2007-02-21), pages 599-607, XP019513691 ISSN: 1432-0614
- IMADA K ET AL: "Assembly characteristics of flagellar cap protein HAP2 of Salmonella: decamer and pentamer in the ph-sensitive equilibrium" JOURNAL OF MOLECULAR BIOLOGY, LONDON, GB, vol. 277, no. 4, 10 April 1998 (1998-04-10), pages 883-891, XP004462458 ISSN: 0022-2836
- HOMMA M ET AL: "Excretion of unassembled flagellin by Salmonella typhimurium mutants deficient in hook-associated proteins." JOURNAL OF BACTERIOLOGY SEP 1984, vol. 159, no. 3, September 1984 (1984-09), pages 1056-1059, XP002522793 ISSN: 0021-9193
- CHEN LEI ET AL: "The Bacillus subtilis sigma-D-dependent operon encoding the flagellar proteins FliD, FliS, and FliT" JOURNAL OF BACTERIOLOGY, vol. 176, no. 11, 1994, pages 3093-3101, XP002522794 ISSN: 0021-9193
- MAJANDER KATARIINA ET AL: "Simultaneous display of multiple foreign peptides in the FliD capping and FliC filament proteins of the Escherichia coli flagellum." APPLIED AND ENVIRONMENTAL MICROBIOLOGY AUG 2005, vol. 71, no. 8, August 2005 (2005-08), pages 4263-4268, XP002522795 ISSN: 0099-2240
- WU SAU-CHING ET AL: "Functional production and characterization of a fibrin-specific single-chain antibody fragment from Bacillus subtilis: Effects of molecular chaperones and a wall-bound protease on antibody fragment production" APPLIED AND ENVIRONMENTAL MICROBIOLOGY, vol. 68, no. 7, July 2002 (2002-07), pages 3261-3269, XP002522796 ISSN: 0099-2240
- BERGER ELDIE ET AL: "Impact of Inactivated Extracellular Proteases on the Modified Flagellin Type III Secretion Pathway of Bacillus halodurans" APPLIED AND ENVIRONMENTAL MICROBIOLOGY, vol. 75, no. 1, January 2009 (2009-01), pages 271-274, XP009114937 ISSN: 0099-2240

### SEQUENCE LISTING

<110> CSIR - Biosciences
<120> Production of Heterologous proteins or peptides
<130> P6993PC00/GSK
<140> PCT/IB2008/055143
   <141> 2008-12-08
<160> 71
<170> PatentIn version 3.3
<210> 1
   <211> 327
   <212> PRT
   <213> Bacillus halodurans
<400> 1
   Met Ile Ile Asn His Asn Leu Pro Ala Met Asn Ala His Arg Asn Met
   1 5 10 15
   Gly Ile Asn Leu Asn Gln Gly Gln Glu Ala Met Glu Lys Leu Ser Ser
   20 25 30
   Gly Leu Arg Ile Asn Arg Ala Gly Asp Asp Ala Ala Gly Leu Ala Ile
   35 40 45
   Ser Glu Lys Met Arg Ala Gln Ile Arg Gly Leu Asp Gln Ala Ser Arg
   50 55 60
   Asn Ser Gln Asp Gly Ile Ser Leu Ile Gln Thr Ala Glu Gly Ala Leu
   65 70 75 80
   Asp Glu Val His Ser Ile Leu Gln Arg Met Arg Glu Leu Ala Val Gln
   85 90 95
   Ser Ser Asn Glu Thr Asn Val Glu Gln Asp Gln Ala Ala Leu Asn Asp
   100 105 110
   Glu Phe Gln Gln Leu Val Glu Glu Ile Glu Arg Ile Lys Asp Thr Thr
   115 120 125
   Gln Phe Asn Thr Gln Lys Leu Leu Asp Asp Thr Val Asp Thr Val Gln
   130 135 140
   Leu Gln Val Gly Ala Asn Ser Gly Glu Leu Ile Glu Leu Asp Leu Thr
   145 150 155 160
   Lys Val Asp Leu Ser Ala Ile His Thr Ala Leu Ala Ala Glu Asp Ile
   165 170 175
   Thr Asp His Thr Asn Ala Gln Ser Ala Ile Asp Ala Ile Asp Glu Gln
   180 185 190
   Leu Lys Ala Val Ser Glu Gly Arg Ser Tyr Leu Gly Ala Met Gln Asn
   195 200 205
   Arg Leu Glu His Thr Ile Lys Asn Leu Asp Asn Ala Ser Glu Asn Leu
   210 215 220
   Gln Ala Ala Glu Ser Arg Ile Arg Asp Val Asp Met Ala Lys Glu Met
   225 230 235 240
   Met Glu Phe Thr Arg Thr Asn Ile Leu Asn Gln Ala Ser Gln Ala Met
   245 250 255
   Leu Ala Gln Ala Asn Gln Gln Pro Gln Ala Val Leu Gln Leu Leu Arg
   260 265 270
   Leu Asp Glu Ser Gly Ala Asp Tyr Lys Asp Asp Asp Asp Lys Gly Gly
   275 280 285
   Val Asp Met Ser Trp Glu Thr Trp Glu Arg Glu Ile Glu Asn Tyr Thr
   290 295 300
   Arg Gln Ile Tyr Arg Ile Ile Glu Glu Ser Gln Glu Gln Gln Asp Arg
   305 310 315 320
   Asn Glu Arg Asp Leu Leu Glu
   325
<210> 2
   <211> 532
   <212> PRT
   <213> Bacillus halodurans
<400> 2
   Met Arg Ile Gly Gly Ile Ala Ser Gly Ile Asp Thr Glu Ser Met Ile
   1               5                   10                  15
   Lys Gln Leu Met Gln Val Glu Arg Ile Pro Leu Asn Lys Phe Thr Gln
               20                  25                  30
   Arg Lys Ile Thr Leu Glu Trp Gln Arg Asp Ala Tyr Arg Glu Val Asn
           35                  40                  45
   Leu Leu Leu Lys Lys Leu Asp Asp Ala Ala Ala Asn Ile Arg Leu Arg
   50 55 60
   Ser Ser Leu Asn Thr Lys Glu Ala Ser Thr Thr Ser Lys Ala Phe Thr
   65 70 75 80
   Ala Gln Pro Asn Ala Gln Val Arg Asn Gly Ser Tyr Gln Leu Lys Val
   85 90 95
   Asn Gln Ile Ala Thr Gln Ser Arg Asn Ile Ser Ser Glu Ala Ile Ser
   100 105 110
   Asn Gly Ser Thr Lys Ile Ser Thr Thr Arg Ala Leu Asn Glu Gln Asn
   115 120 125
   Val Tyr Ala Asp Gly Leu Asn Ile Glu Asp Tyr His Gly Gln Thr Phe
   130 135 140
   Thr Ile Thr Thr Tyr Asn Ser Ser Gly Ala Ala Val Glu Lys Ser Phe
   145 150 155 160
   Thr Ile Asp Thr Ser Lys Ser Leu Asp Ser Leu Phe Lys Asp Ile Asn
   165 170 175
   Ser Ala Gly Leu Gly Val Arg Met Ser Tyr Asn Ser Thr Tyr Asp Lys
   180 185 190
   Val Ile Ile Glu Arg Thr Glu Thr Gly Ala Phe Asn Ala Ala Asp Gly
   195 200 205
   Ser Asn Asp Tyr Gln Ile Val Phe Gly Gly Asp Thr Gly Phe Leu Asn
   210 215 220
   Asp Val Leu Lys Leu Asn Gln Ala Asn Glu Val Ser Gly Thr Asn Ala
   225 230 235 240
   Glu Val Glu Phe Ile Asp Pro Ile Met Ser Ser Glu Pro Ile Val Val
   245 250 255
   Ser Asp Ser Arg Thr Asn Arg Val Thr Val Gly Gly Ile Thr Phe Ser
   260 265 270
   Leu Thr Gly Thr Thr Glu Gly Phe Glu Thr Leu Asn Val Ser Ser Asn
   275 280 285
   Thr Asp Ala Ala Phe Glu Lys Val Met Glu Phe Val Asp Thr Tyr Asn
   290 295 300
   Ala Thr Ile Thr Glu Leu Arg Ser Leu Leu Ser Glu Pro Arg Tyr Arg
   305                 310                 315                 320
   Asp Tyr Pro Pro Leu Thr Glu Glu Gln Arg Arg Glu Leu Ser Glu Arg
                   325                 330                 335
   Glu Ala Glu Leu Trp Asp Glu Lys Ala Lys Ser Gly Leu Leu Arg Asn
               340                 345                 350
   Asp Ser Met Leu Asn Ser Leu Leu Ala Gln Met Arg Ala Asp Leu Tyr
           355                 360                 365
   Ala Pro Val Gln Thr Asn Gly Gln Phe Ser Ser Ile Thr Gln Ile Gly
       370                 375                 380
   Ile Thr Thr Ser Ser Asp Tyr Arg Leu Gly Gly Phe Leu Glu Val Asp
   385                 390                 395                 400
   Glu Asp Lys Leu Arg Ala Ala Leu Glu Ala Asp Pro Asp Ser Val His
                   405                 410                 415
   Gln Leu Leu Asn Gly Thr Ala Asn Ser Ser Leu Thr Ser Ile Pro Val
               420                 425                 430
   Lys Asp Arg Thr Ser Gln Gln Arg Ser Glu Ile Tyr Ser Gln Thr Gly
           435                 440                 445
   Leu Val Gly Arg Ile Arg Ser Ser Leu Ser Ser Thr Met Asn Asp Ile
       450                 455                 460
   Val Ala Arg Ala Gly Asn Glu Arg Arg Thr Glu Gln Gln Phe Thr Ile
   465                 470                 475                 480
   Gly Arg Gln Ile Leu Asp Val Asp Lys Arg Ile Asp His Phe Gln Gln
                   485                 490                 495
   Arg Leu Ile Gln Ile Glu Asn Arg Tyr Trp Ala Gln Phe Ser Arg Met
               500                 505                 510
   Glu Gln Met Met Asn Gln Ala Asn Ala Gln Tyr Ala Ser Leu Gln Gln
           515                 520                 525
   Phe Phe Val Thr
       530
<210> 3
   <211> 372
   <212> PRT
   <213> Bacillus halodurans
<400> 3
   Met Lys Arg Arg Val His Val Leu Ile Met Thr Ile Leu Met Ile Val
   1               5                   10                  15
   Leu Ala Thr Gly Thr Ala Phe Ala Asp Asn Arg Glu Asp Thr Glu Asp
               20                  25                  30
   Thr Glu Glu Tyr Leu Val Gly Phe Lys Asn Glu Ala Ala Val Gln Ala
           35                  40                  45
   Phe Ser Asn Asn Val Thr Thr Ser Ala Val Glu Val Gln His Glu Tyr
       50                  55                  60
   Glu Asn Leu Pro Val Ile Val Ser Glu Leu Ser Thr Glu Val Ala Gln
   65                  70                  75                  80
   Leu Leu Glu Asn Asp Pro Ser Val Glu Phe Ile Glu Lys Asn Glu Arg
                   85                  90                  95
   Val Tyr Leu Asp Pro Leu Val Met Asn Asn Ile Gln Glu Thr Asp Ile
               100                 105                 110
   Pro Lys Leu Glu Glu Arg Met Lys Arg Gly Asp Gly Val Lys Ile Ala
           115                 120                 125
   Val Leu Asp Thr Gly Ile Ala Ser His Asp Asp Leu His Val Ile Asp
       130                 135                 140
   Gly Val Ser Phe Val Ser Val Glu Pro Phe Tyr Arg Asp Leu Asn Gly
   145                 150                 155                 160
   His Gly Thr His Val Ala Gly Thr Ile Ala Ala Gln Glu Asn Asp Glu
                   165                 170                 175
   Ala Ser Thr Gly Ile Ala Pro Asn Val Glu Leu Tyr Ala Val Lys Val
               180                 185                 190
   Leu Asn Gly Leu Gly Ala Gly Ser Ile Ala Ser Ile Thr Asn Gly Val
           195                 200                 205
   Asp Trp Ala Ile Ser Asn Asp Met Asp Ile Ile Asn Met Ser Leu Gly
       210                 215                 220
   Thr Asn Thr Asp Ser Glu Ala Leu Gln Arg Ala Val Glu Arg Ala His
   225                 230                 235                 240
   Asp His Gly Ile Leu Ile Ile Ala Ala Ala Gly Asn Ser Gly Glu Ala
   245 250 255
   Asp Lys Gln His Thr Ile Asp Tyr Pro Ala Arg Tyr Asp Ser Val Val
   260 265 270
   Ala Val Gly Ala Val Asp Gly Asn Asn Glu Arg Ala Ser Phe Ser Ser
   275 280 285
   Tyr Gly Glu Gln Leu Glu Ile Met Ala Pro Gly Val Glu Ile His Ser
   290 295 300
   Thr Phe Leu Phe Asn Arg Tyr Glu Arg Leu Ser Gly Thr Ser Met Ala
   305 310 315 320
   Ser Pro His Val Thr Gly Ala Ala Ala Leu Ile Lys Ser Asn Asn Pro
   325 330 335
   Glu Leu Thr Asn Glu Gln Ile Arg Lys Arg Leu Asn Thr Thr Ala Thr
   340 345 350
   Pro Leu Gly Asn Pro Phe Tyr Tyr Gly His Gly Leu Leu Asn Val Asp
   355 360 365
   Ala Ala Leu Asp
   370
<210> 4
   <211> 792
   <212> PRT
   <213> Bacillus halodurans
<400> 4
   Met Asn Lys Arg Leu Lys Arg Trp Ser Ala Ile Met Ser Ile Val Leu
   1 5 10 15
   Leu Val Asn Leu Leu Val Pro Val Gly Thr Phe Ala Asp Thr Asp Glu
   20 25 30
   Glu Glu Lys Val Asp Ile Ile Val Thr Tyr Lys Asp Val Val Ser Glu
   35 40 45
   Ser Pro Asp Ser Arg Leu Ser Val Tyr Glu Asn Glu Gln Thr Met Lys
   50 55 60
   Thr Leu Pro Ile Lys Thr Met Thr Val Pro Val Ser Glu Val Glu Arg
   65 70 75 80
   Leu Lys Glu Asp Pro Asn Val Val Ser Val Ser Leu Asp Gln Pro Leu
                   85                  90                  95
   Gln Leu Met Ser Asp Thr Arg Glu Leu Gly Glu His Asp Trp Asn Asn
               100                 105                 110
   Asp Met Val Lys Ala Phe Asp Ala Trp Asp Asp Gly Tyr Thr Gly Lys
           115                 120                 125
   Gly Val Lys Val Ala Val Phe Asp Thr Gly Phe Asp Gly His Gln Asp
       130                 135                 140
   Ile Thr Tyr Ala Gly Gly His Ser Val Phe Glu Gly Glu Pro Tyr Thr
   145                 150                 155                 160
   His Asp His His Gly His Gly Thr His Val Ala Gly Ile Ile Gly Ala
                   165                 170                 175
   Arg Glu Gly Thr Leu His Gln Gly Ile Ala Pro Asp Val Gln Leu Tyr
               180                 185                 190
   Gly Val Lys Val Phe Ser Gln Glu Lys Gly Gly Asn Thr Ser Asp Leu
           195                 200                 205
   Ile Ala Gly Ile Asp Trp Ala Ile Gln Glu Gly Met Asp Ile Ile Asn
       210                 215                 220
   Met Ser Leu Gly Tyr Thr Asn Glu Val Pro Ala Val His Thr Ala Ile
   225                 230                 235                 240
   Lys Gln Ala Val Ala Gln Glu Ile Leu Val Val Ala Ala Ser Gly Asn
                   245                 250                 255
   Gly Gly Lys Ala Asp Gly Ser Gly Glu Thr Ile Glu Tyr Pro Ala Lys
               260                 265                 270
   Tyr Asp Glu Val Ile Ala Val Ala Ser Val Asp Lys Glu Met Lys Arg
           275                 280                 285
   Thr Asn Thr Ser Ala Thr Gly Val Glu Asn Glu Leu Ala Ala Pro Gly
       290                 295                 300
   His Leu Ile Gly Gly Leu Ala Pro Gly Asn Lys Tyr Val Phe Met Ser
   305                 310                 315                 320
   Gly Thr Ser Gln Ala Thr Pro His Val Thr Ser Leu Ala Ala Ile Leu
                   325                 330                 335
   Met Gly Lys His Pro Glu Leu Ser Ser Gln Gln Ile Arg Ala Leu Leu
               340                 345                 350
   Val Glu Gln Ser Leu Asp Leu Gly Ser Glu Gly His Asp Arg Leu Tyr
           355                 360                 365
   Gly Tyr Gly Leu Ala Gln Tyr Val Ser Ser Thr Pro Pro Asp Glu Glu
       370                 375                 380
   Glu Asn Glu Glu Ser Pro Ala Glu Asn Pro Gln Glu Gln Pro Ser Asp
   385                 390                 395                 400
   Gly Lys Glu Asn Glu Gly Ser Glu Asp Gln Gly Ser Thr Pro Pro Asp
                   405                 410                 415
   Glu Glu Glu Asn Glu Glu Ser Pro Ala Glu Asp Pro Gln Glu Gln Pro
               420                 425                 430
   Ser Asp Gly Lys Glu Asn Lys Gly Ser Glu Asn Gln Gly Ser Thr Pro
           435                 440                 445
   Pro Asp Glu Glu Glu Asn Glu Glu Ser Pro Ala Glu Val Pro Gln Glu
       450                 455                 460
   Gln Pro Ser Asp Gly Lys Glu Asn Glu Gly Ser Glu Asp Gln Gly Ser
   465                 470                 475                 480
   Thr Pro Pro Asp Glu Glu Glu Asn Glu Glu Ser Pro Ala Glu Asp Pro
                   485                 490                 495
   Gln Glu Gln Pro Ser Asp Gly Lys Glu Asn Glu Glu Ser Lys Asn Pro
               500                 505                 510
   Asp Ser Ala Pro Pro Ala Gly Glu Lys Glu Gly Lys Gln Thr Ala Arg
           515                 520                 525
   Val Gln Val Lys Pro Val Asn Leu Gly Gly Val Ala Ile Val Ser Asn
       530                 535                 540
   Ala Asp Val Ala Ser Val Leu Asp Asn Gly Thr Leu Val Val Phe Phe
   545                 550                 555                 560
   Asp Ser Ala Leu Asp Asp Leu Thr Arg Leu Ala Leu Thr Ala Asp Gln
                   565                 570                 575
   Val Lys Glu Leu Lys Asp Arg Gly Ile Thr Leu Val Ile Ala Lys His
   580 585 590
   Asp Leu Glu Leu Val Ile Pro His Gly Val Phe Lys Ala Gly Asp Val
   595 600 605
   Val Ile Glu Phe Glu Arg Val Val Gly Lys Gly Ile Pro Tyr Ala Gly
   610 615 620
   Gln Ala Lys Ser Thr Val Tyr Gln Phe Lys Ile Ile Gln Asn Gly Gln
   625 630 635 640
   Gln Val Arg His Phe Asp Glu Glu Ile Glu Met Gly Phe Arg Val Asp
   645 650 655
   Gln Glu Lys Asn Val Asn Asn Leu Lys Ile Tyr Tyr Trp Asn Glu Ser
   660 665 670
   Leu Asn Glu Trp Glu Lys Ile Gly Gly Asn Tyr Gln Glu Gly Phe Ile
   675 680 685
   Val Ala Arg Thr Asn His Phe Ser Thr Tyr Thr Val Phe Asp Ser Ala
   690 695 700
   Val Phe Glu Glu Thr Lys Ser Gly Thr Pro Ile Ser Gly Gly Lys Thr
   705 710 715 720
   Asn Gly Asn Ser Thr Thr Glu Gly Thr Thr Asn Arg Gly Thr Ser Lys
   725 730 735
   Asn Gly Thr Gly Ser Glu Pro Gln Ala Glu Glu Ser Asn Glu Gln Asn
   740 745 750
   Asn Lys Asp Gly Thr Leu Pro Lys Thr Ala Thr Asn Leu Tyr Asn Ser
   755 760 765
   Leu Ala Ile Gly Ala Leu Leu Leu Leu Ile Gly Phe Val Leu Leu Arg
   770 775 780
   Lys Ser Lys Arg Arg Ile Val Glu
   785 790
<210> 5
   <211> 799
   <212> PRT
   <213> Bacillus halodurans
<400> 5
   Met Met Met Arg Thr Phe Arg Thr Phe Val Ser Phe Thr Leu Ile Leu
   1               5                   10                  15
   Leu Leu Thr Leu Thr Ser Tyr Ile Pro Val Ser Gln Ala Asn Gly Glu
               20                  25                  30
   Ser Pro Ser Leu Ala Pro Met Val Gly Glu Ile Asp Thr Ser Ser Ser
           35                  40                  45
   Ala Leu Glu Thr Val Ile Val Glu Ile Glu Asp Pro Ser Ile Ile Glu
       50                  55                  60
   Ala Lys His Gln Gly Gln Lys Gln Ser Lys Asn Glu Leu Lys Gln Ala
   65                  70                  75                  80
   Arg Gln Ser Val Ile Glu Gln Ile Gln Asp Leu Val Pro Ser Ser Thr
                   85                  90                  95
   Val Thr His Glu Tyr Asp Phe Leu Phe Ser Gly Phe Ala Leu Glu Leu
               100                 105                 110
   Pro Ala His Gln Ile Pro Ser Ile Leu Gly Ile Asp Gly Val His Ala
           115                 120                 125
   Val Tyr Pro Asn Ile Glu Tyr Glu Val Glu Thr Asp Phe Asp Glu Val
       130                 135                 140
   Val Ile Glu Lys Asp Ala Tyr Ser Pro Glu Met Leu Asp Ser Ala Pro
   145                 150                 155                 160
   Phe Ile Gly Ala Asn Asp Ala Trp Glu Ala Gly Tyr Thr Gly Glu Gly
                   165                 170                 175
   Ile Thr Val Ala Ile Leu Asp Thr Gly Val Asp Tyr Thr His Pro Asp
               180                 185                 190
   Leu Val His Ala Phe Gly Asp Tyr Lys Gly Trp Asp Phe Ile Asp Asn
           195                 200                 205
   Asn Asp Asp Pro Gln Glu Thr Pro Pro Gly Asp Pro Arg Gly Ile Glu
       210                 215                 220
   Thr Thr His Gly Thr His Val Ala Gly Thr Val Ala Ala Asn Gly Leu
   225                 230                 235                 240
   Ile Lys Gly Val Ala Pro Asp Ala Asn Leu Leu Ala Tyr Arg Val Leu
                   245                 250                 255
   Gly Pro Gly Gly Arg Gly Ser Thr Ala Gly Val Ile Ala Gly Ile Glu
               260                 265                 270
   Arg Ala Val Gln Asp Gly Ala Asp Ile Met Asn Leu Ser Leu Gly Asn
           275                 280                 285
   Thr Leu Asn Asp Pro Asp Phe Ala Thr Ser Ile Ala Leu Asp Trp Ala
       290                 295                 300
   Met Ala Glu Gly Val Val Ala Val Thr Ser Asn Gly Asn Ser Gly Pro
   305                 310                 315                 320
   Asn Asn Trp Thr Val Gly Ser Pro Gly Thr Ser Arg Asp Ala Ile Ser
                   325                 330                 335
   Val Gly Ala Thr Arg Leu Pro Tyr Asn Lys Tyr Lys Ala Ser Val Phe
               340                 345                 350
   Thr Ser Asp Gly Ile Asp Tyr Pro Ser Ala Asp Ile Met Gly Phe Pro
           355                 360                 365
   Ser Asp Glu Glu Leu Leu Glu Leu Asp Gly Glu Thr Tyr Glu Tyr Ala
       370                 375                 380
   Phe Ala Gly Leu Gly Lys Pro Gly Asp Phe Glu Gly Val Asp Val Glu
   385                 390                 395                 400
   Gly Lys Ile Ala Leu Ile Val Arg Gly Glu Ile Pro Phe Val Glu Lys
                   405                 410                 415
   Ala Glu Asn Ala Lys Ala Ala Gly Ala Val Gly Ala Ile Ile Tyr Asn
               420                 425                 430
   Asn Val Ala Gly Val Gln Pro Thr Val Pro Gly Leu Ala Ile Pro Thr
           435                 440                 445
   Ile Met Leu Ser Asn Glu Asp Gly Leu Lys Met Arg Asn Glu Leu Glu
       450                 455                 460
   Asn Gly Gln Asn Thr Val Thr Phe Ser Ile Glu Phe Asp Lys Leu Val
   465                 470                 475                 480
   Gly Glu Thr Val Ala Asp Phe Ser Ser Arg Gly Pro Val Met His Thr
                   485                 490                 495
   Trp Met Ile Lys Pro Asp Val Ser Ala Pro Gly Val Ala Ile Val Ser
               500                 505                 510
   Thr Ile Pro Thr His Gln Pro Asp Asp Pro Tyr Gly Tyr Gly Ser Arg
           515                 520                 525
   Gln Gly Thr Ser Met Ala Ser Pro His Val Ala Gly Ala Ala Ala Leu
       530                 535                 540
   Leu Leu Glu Ala His Pro Asn Trp Gly Val Asp His Val Lys Ala Ala
   545                 550                 555                 560
   Leu Met Asn Thr Ala Glu Asn Leu Val Asp Glu Asn Gly Asn Arg Tyr
                   565                 570                 575
   Pro His Asn Thr Gln Gly Ala Gly Ser Ile Arg Ile Val Asp Ala Ile
               580                 585                 590
   Glu Ser Glu Thr Leu Val Thr Pro Gly Ser His Ser Phe Gly Thr Phe
           595                 600                 605
   Thr Lys Glu Arg Gly Lys Gln Val Glu Arg Gln His Phe Thr Ile His
       610                 615                 620
   Asn Leu Ser Asn Lys Arg Lys Thr Tyr Gln Phe Asp Val Gln Phe Ala
   625                 630                 635                 640
   Gly Asn Pro Asp Gly Ile Lys Val Lys Thr Ser Lys Asn Leu Arg Val
                   645                 650                 655
   Gln Pro Gly Lys Thr Gln Lys Ile Asn Phe Asn Val Gln Val Asp Ala
               660                 665                 670
   Arg Lys Leu Asp Pro Gly Tyr Tyr Glu Gly Thr Ile Ile Val Ser Asp
           675                 680                 685
   Gly Ser Gln Thr Val Glu Val Pro Thr Ile Leu Phe Val Ser Glu Pro
       690                 695                 700
   Asp Tyr Pro Arg Val Thr Thr Phe Asp Leu Asp Ile Asp Glu Asn Gly
   705                 710                 715                 720
   Val Leu Phe Gly Ser Ala Tyr Leu Pro Asn Gly Ala Glu Glu Phe Gly
                   725                 730                 735
   Leu Trp Ile Tyr Thr Arg Asp Thr Leu Glu Tyr Val Thr Ser Ala Ala
               740                 745                 750
   Val Val Gln Asn Ile Gly Lys Gly Tyr His Asp Val Thr Trp Asp Thr
           755                 760                 765
   Thr Glu Leu Ala Pro Gly Arg Tyr Tyr Val Phe Ala Tyr Ala Glu Arg
   770 775 780
   Leu Gly Lys Thr Asp Phe Val Leu Gly Asp Glu Ile Val Val Lys
   785 790 795
<210> 6
   <211> 301
   <212> PRT
   <213> Bacillus halodurans
<400> 6
   Pro Val Ile His Ala Glu Leu Thr Lys Lys Glu Leu Lys Lys Leu Lys
   1 5 10 15
   Lys Asp Pro Asn Val Lys Ala Ile Glu Lys Asn Ala Glu Val Thr Ile
   20 25 30
   Ser Gln Thr Val Pro Trp Gly Ile Ser Phe Ile Asn Thr Gln Gln Ala
   35 40 45
   His Asn Arg Gly Ile Phe Gly Asn Gly Ala Arg Val Ala Val Leu Asp
   50 55 60
   Thr Gly Ile Ala Ser His Pro Asp Leu Arg Ile Ala Gly Gly Ala Ser
   65 70 75 80
   Phe Ile Ser Ser Glu Pro Ser Tyr His Asp Asn Asn Gly His Gly Thr
   85 90 95
   His Val Ala Gly Thr Ile Ala Ala Leu Asn Asn Ser Ile Gly Val Leu
   100 105 110
   Gly Val Ala Pro Ser Ala Asp Leu Tyr Ala Val Lys Val Leu Asp Arg
   115 120 125
   Asn Gly Ser Gly Ser Leu Ala Ser Val Ala Gln Gly Ile Glu Trp Ala
   130 135 140
   Ile Asn Asn Asn Met His Ile Ile Asn Met Ser Leu Gly Ser Thr Ser
   145 150 155 160
   Gly Ser Ser Thr Leu Glu Leu Ala Val Asn Arg Ala Asn Asn Ala Gly
   165 170 175
   Ile Leu Leu Val Gly Ala Ala Gly Asn Thr Gly Arg Gln Gly Val Asn
   180 185 190
   Tyr Pro Ala Arg Tyr Ser Gly Val Met Ala Val Ala Ala Val Asp Gln
           195                 200                 205
   Asn Gly Gln Arg Ala Ser Phe Ser Thr Tyr Gly Pro Glu Ile Glu Ile
       210                 215                 220
   Ser Ala Pro Gly Val Asn Val Asn Ser Thr Tyr Thr Gly Asn Arg Tyr
   225                 230                 235                 240
   Val Ser Leu Ser Gly Thr Ser Met Ala Thr Pro His Val Ala Gly Val
                   245                 250                 255
   Ala Ala Leu Val Lys Ser Arg Tyr Pro Ser Tyr Thr Asn Asn Gln Ile
               260                 265                 270
   Arg Gln Arg Ile Asn Gln Thr Ala Thr Tyr Leu Gly Ser Pro Ser Leu
           275                 280                 285
   Tyr Gly Asn Gly Leu Val His Ala Gly Arg Ala Thr Gln
       290                 295                 300
<210> 7
   <211> 819
   <212> DNA
   <213> Bacillus halodurans
<400> 7
   atgattatca atcacaattt accagcaatg aatgcgcatc gtaacatggg tatcaatctt 60
   aaccaaggtc aagaagcgat ggagaagctt tcttcaggtc ttcgcattaa ccgtgcagga 120
   gacgatgctg caggtcttgc catctctgaa aaaatgcgtg cgcaaatccg tggtttggat 180
   caagcgtctc gtaactcaca agacggtatt tcgttaattc aaacagctga aggtgcgctt 240
   gatgaagtac attctattct tcaacgtatg cgtgagctag cggttcaatc ttcgaacgaa 300
   acgaatgttg agcaagatca agcagctctt aacgatgaat tccaacaatt agttgaggaa 360
   attgaaagaa tcaaagatac aactcaattt aatacgcaaa aattactcga tgatacagta 420
   gatactgtac aacttcaagt tggtgctaat tctggtgaat taattgaact tgatttaaca 480
   aaagttgatt tatcagctat ccatacagct ttggcggctg aggatattac tgaccacact 540
   aatgcacaat cagctattga cgctattgat gagcaattaa aagctgtttc agaaggtcgc 600
   tcttacctag gagctatgca aaaccgccta gagcatacaa tcaaaaacct tgataatgct 660
   tctgaaaacc ttcaagctgc tgagtctcgt atccgtgacg tagacatggc gaaagaaatg 720
   atggagttca caagaacaaa catcttaaac caagcgtctc aagcgatgct tgctcaagca 780
   aaccaacagc cacaagctgt attacaatta cttcgttaa 819
<210> 8
   <211> 25
   <212> DNA
   <213> Artificial Sequence
<220>
   <223> PCR primer
<400> 8
   cttggtaccg cgtgggaatg ttgca 25
<210> 9
   <211> 27
   <212> DNA
   <213> Artificial Sequence
<220>
   <223> PCR primer
<400> 9
   cttggatcct gcacttctac cgctgag 27
<210> 10
   <211> 26
   <212> DNA
   <213> Artificial Sequence
<220>
   <223> PCR primer
<400> 10
   cttggatccg gcttcacctc atgtga 26
<210> 11
   <211> 26
   <212> DNA
   <213> Artificial Sequence
<220>
   <223> PCR primer
<400> 11
   ctcccgggtg gttgtcacag cagcgg 26
<210> 12
   <211> 31
   <212> DNA
   <213> Artificial Sequence
<220>
   <223> PCR primer
<400> 12
   gcaggtaccg ttggtgttca agatgtttac g 31
<210> 13
   <211> 31
   <212> DNA
   <213> Artificial Sequence
<220>
   <223> PCR primer
<400> 13
   gcaggatcca ggcgttgctt gagacgtacc a 31
<210> 14
   <211> 30
   <212> DNA
   <213> Synthetic
<400> 14
   gcaggatccg gacaggaagc gaacctcaag 30
<210> 15
   <211> 30
   <212> DNA
   <213> Artificial Sequence
<220>
   <223> PCR primer
<400> 15
   gcacccgggc aagtcctaga gtacaataac 30
<210> 16
   <211> 27
   <212> DNA
   <213> Artificial Sequence
<220>
   <223> PCR primer
<400> 16
   cgtgttaccg atgtgtagtg ccttatc 27
<210> 17
   <211> 30
   <212> DNA
   <213> Artificial Sequence
<220>
   <223> PCR primer
<400> 17
   cttggatcct tcatacgtct cgccatcgag 30
<210> 18
   <211> 28
   <212> DNA
   <213> Artificial Sequence
<220>
   <223> PCR primer
<400> 18
   cgtggatccc gaaggtacga tcatcgta 28
<210> 19
   <211> 32
   <212> DNA
   <213> Artificial Sequence
<220>
   <223> PCR primer
<400> 19
   gtcccgggaa gcacgagtgg attcatggta ta 32
<210> 20
   <211> 31
   <212> DNA
   <213> Artificial Sequence
<220>
   <223> PCR primer
<400> 20
   gtaggtaccc tcgatgcgaa agttctcgat g 31
<210> 21
   <211> 30
   <212> DNA
   <213> Artificial Sequence
<220>
   <223> PCR primer
<400> 21
   gcaggatccg taccagccac gtgagttccg 30
<210> 22
   <211> 31
   <212> DNA
   <213> Artificial Sequence
<220>
   <223> PCR primer
<400> 22
   gcaggatccg ctagatactc tggtgttatg g 31
<210> 23
   <211> 31
   <212> DNA
   <213> Artificial Sequence
<220>
   <223> PCR primer
<400> 23
   gatcccgggc ctcctatcat acccaaatga g 31
<210> 24
   <211> 30
   <212> DNA
   <213> Artificial Sequence
<220>
   <223> PCR primer
<400> 24
   cgaggatccc gtatttaaag aggaacgtaa 30
<210> 25
   <211> 29
   <212> DNA
   <213> Artificial Sequence
<220>
   <223> PCR primer
<400> 25
   cgaggatccc gagcagtgat tacagattg 29
<210> 26
   <211> 31
   <212> DNA
   <213> Artificial Sequence
<220>
   <223> PCR primer
<400> 26
   cgacccgggc agagagctca ttatgcttct c 31
<210> 27
   <211> 26
   <212> DNA
   <213> Artificial Sequence
<220>
   <223> PCR primer
<400> 27
   cttagatcat ggttagaatc aagagg 26
<210> 28
   <211> 26
   <212> DNA
   <213> Artificial Sequence
<220>
   <223> PCR primer
<400> 28
   gcttgtgctg ggcaaaggag gcgaag 26
<210> 29
   <211> 30
   <212> DNA
   <213> Artificial Sequence
<220>
   <223> PCR primer
<400> 29
   ctcggtaccc tcgcgttacg ctctttctgt 30
<210> 30
   <211> 26
   <212> DNA
   <213> Artificial Sequence
<220>
   <223> PCR primer
<400> 30
   ctcctcgagc gaccttctga aacagc 26
<210> 31
   <211> 17
   <212> DNA
   <213> Artificial Sequence
<220>
   <223> PCR primer
<400> 31
   tgaccggcag caaaatg 17
<210> 32
   <211> 21
   <212> DNA
   <213> Artificial Sequence
<220>
   <223> PCR primer
<400> 32
   caacaaagta acggttgagc g 21
<210> 33
   <211> 30
   <212> DNA
   <213> Artificial Sequence
<220>
   <223> PCR primer
<400> 33
   cgaggatcca agaccggcag agttaatgtc 30
<210> 34
   <211> 45
   <212> DNA
   <213> Artificial Sequence
<220>
   <223> PCR primer
<400> 34
   cacgtcgact cgagcccggg atcctttaat acgcaaaaat tactc 45
<210> 35
   <211> 51
   <212> DNA
   <213> Artificial Sequence
<220>
   <223> PCR primer
<400> 35
   cacgtcgact cgagcccggg atggatccag aatgcacaat cagctattga c 51
<210> 36
   <211> 30
   <212> DNA
   <213> Artificial Sequence
<220>
   <223> PCR primer
<400> 36
   gacgtcgaca gtgtggtcag taatatcctc 30
<210> 37
   <211> 32
   <212> DNA
   <213> Artificial Sequence
<220>
   <223> PCR primer
<400> 37
   cgacccgggg aagaagctga agacgatgca gc 32
<210> 38
   <211> 26
   <212> DNA
   <213> Artificial Sequence
<220>
   <223> PCR primer
<400> 38
   cgaggtacca ggagtttgtc cttctg 26
<210> 39
   <211> 29
   <212> DNA
   <213> Artificial Sequence
<220>
   <223> PCR primer
<400> 39
   cgtcaggatc cttacataaa ccaattcca 29
<210> 40
   <211> 11
   <212> DNA
   <213> Artificial Sequence
<220>
   <223> PCR primer
<400> 40
   gtctaggatc c 11
<210> 41
   <211> 30
   <212> DNA
   <213> Artificial Sequence
<220>
   <223> PCR primer
<400> 41
   gacggatcct ttgcttccat ttaaagatct 30
<210> 42
   <211> 32
   <212> DNA
   <213> Artificial Sequence
<220>
   <223> PCR primer
<400> 42
   gtgctgcagg tatttaaaga ggaacgtaaa cg 32
<210> 43
   <211> 32
   <212> DNA
   <213> Artificial Sequence
<220>
   <223> PCR primer
<400> 43
   ctcgaggatc cttattctaa taaatcacgt tc 32
<210> 44
   <211> 301
   <212> PRT
   <213> Bacillus halodurans
<220>
   <221> misc_feature
   <222> (181)..(209)
   <223> FliC polypepitde incorporating the HIV immunogenic peptide (aa 181 - 209)
<220>
   <221> misc_feature
   <222> (181)..(209)
   <223> FliC polypepitde incorporating the HIV-antigenic peptide (aa 181 - 209)
<400> 44
   Met Ile Ile Asn His Asn Leu Pro Ala Met Asn Ala His Arg Asn Met
   1               5                   10                  15
   Gly Ile Asn Leu Asn Gln Gly Gln Glu Ala Met Glu Lys Leu Ser Ser
               20                  25                  30
   Gly Leu Arg Ile Asn Arg Ala Gly Asp Asp Ala Ala Gly Leu Ala Ile
           35                  40                  45
   Ser Glu Lys Met Arg Ala Gln Ile Arg Gly Leu Asp Gln Ala Ser Arg
       50                  55                  60
   Asn Ser Gln Asp Gly Ile Ser Leu Ile Gln Thr Ala Glu Gly Ala Leu
   65                  70                  75                  80
   Asp Glu Val His Ser Ile Leu Gln Arg Met Arg Glu Leu Ala Val Gln
                   85                  90                  95
   Ser Ser Asn Glu Thr Asn Val Glu Gln Asp Gln Ala Ala Leu Asn Asp
               100                 105                 110
   Glu Phe Gln Gln Leu Val Glu Glu Ile Glu Arg Ile Lys Asp Thr Thr
           115                 120                 125
   Gln Phe Asn Thr Gln Lys Leu Leu Asp Asp Thr Val Asp Thr Val Gln
       130                 135                 140
   Leu Gln Val Gly Ala Asn Ser Gly Glu Leu Ile Glu Leu Asp Leu Thr
   145                 150                 155                 160
   Lys Val Asp Leu Ser Ala Ile His Thr Ala Leu Ala Ala Glu Asp Ile
                   165                 170                 175
   Thr Asp His Thr Val Asp Thr Arg Pro Asn Asn Asn Thr Arg Lys Ser
               180                 185                 190
   Ile Arg Ile Gly Pro Gly Gln Thr Phe Tyr Ala Thr Gly Asp Trp Ile
           195                 200                 205
   Gln Asn Ala Gln Ser Ala Ile Asp Ala Ile Asp Glu Gln Leu Lys Ala
               210                 215                 220
   Val Ser Glu Gly Arg Ser Tyr Leu Gly Ala Met Gln Asn Arg Leu Glu
   225                 230                 235                 240
   His Thr Ile Lys Asn Leu Asp Asn Ala Ser Glu Asn Leu Gln Ala Ala
                   245                 250                 255
   Glu Ser Arg Ile Arg Asp Val Asp Met Ala Lys Glu Met Met Glu Phe
               260                 265                 270
   Thr Arg Thr Asn Ile Leu Asn Gln Ala Ser Gln Ala Met Leu Ala Gln
           275                 280                 285
   Ala Asn Gln Gln Pro Gln Ala Val Leu Gln Leu Leu Arg
       290                 295                 300
<210> 45
   <211> 906
   <212> DNA
   <213> Bacillus halodurans
<220>
   <221> misc_feature
   <222> (541)..(627)
   <223> FliC nucleotide sequence incorporating the nucleotide sequence encoding the HIV immunogenic peptide (bp 541 - 627)
<220>
   <221> misc feature
   <222> (541)..(627)
   <223> FliC nucleotide sequence incorporating the nucleotide sequence encoding the HIV-antigenic peptide (bp 541 - 627)
<400> 45
   atgattatca atcacaattt accagcaatg aatgcgcatc gtaacatggg tatcaatctt     60
   aaccaaggtc aagaagcgat ggagaagctt tcttcaggtc ttcgcattaa ccgtgcagga    120
   gacgatgctg caggtcttgc catctctgaa aaaatgcgtg cgcaaatccg tggtttggat    180
   caagcgtctc gtaactcaca agacggtatt tcgttaattc aaacagctga aggtgcgctt    240
   gatgaagtac attctattct tcaacgtatg cgtgagctag cggttcaatc ttcgaacgaa    300
   acgaatgttg agcaagatca agcagctctt aacgatgaat tccaacaatt agttgaggaa    360
   attgaaagaa tcaaagatac aactcaattt aatacgcaaa aattactcga tgatacagta    420
   gatactgtac aacttcaagt tggtgctaat tctggtgaat taattgaact tgatttaaca    480
   aaagttgatt tatcagctat ccatacagct ttggcggctg aggatattac tgaccacact    540
   gtcgacacgc gtccaaataa taatacgcgt aaatcaattc gtattggacc aggacaaacg    600
   ttttatgcaa cgggagattg gatccagaat gcacaatcag ctattgacgc tattgatgag    660
   caattaaaag ctgtttcaga aggtcgctct tacctaggag ctatgcaaaa ccgcctagag    720
   catacaatca aaaaccttga taatgcttct gaaaaccttc aagctgctga gtctcgtatc    780
   cgtgacgtag acatggcgaa agaaatgatg gagttcacaa gaacaaacat cttaaaccaa    840
   gcgtctcaag cgatgcttgc tcaagcaaac caacagccac aagctgtatt acaattactt    900
   cgttaa                                                               906
<210> 46
   <211> 308
   <212> PRT
   <213> Bacillus halodurans
<220>
   <221> MISC_FEATURE
   <222> (181)..(216)
   <223> FliC polypeptide sequence incorporating the FLAG::Indolicidin polypeptide sequence (aa 181 - 216)
<400> 46
   Met Ile Ile Asn His Asn Leu Pro Ala Met Asn Ala His Arg Asn Met
   1               5                   10                  15
   Gly Ile Asn Leu Asn Gln Gly Gln Glu Ala Met Glu Lys Leu Ser Ser
               20                  25                  30
   Gly Leu Arg Ile Asn Arg Ala Gly Asp Asp Ala Ala Gly Leu Ala Ile
           35                  40                  45
   Ser Glu Lys Met Arg Ala Gln Ile Arg Gly Leu Asp Gln Ala Ser Arg
       50                  55                  60
   Asn Ser Gln Asp Gly Ile Ser Leu Ile Gln Thr Ala Glu Gly Ala Leu
   65                  70                  75                  80
   Asp Glu Val His Ser Ile Leu Gln Arg Met Arg Glu Leu Ala Val Gln
                   85                  90                  95
   Ser Ser Asn Glu Thr Asn Val Glu Gln Asp Gln Ala Ala Leu Asn Asp
               100                 105                 110
   Glu Phe Gln Gln Leu Val Glu Glu Ile Glu Arg Ile Lys Asp Thr Thr
           115                 120                 125
   Gln Phe Asn Thr Gln Lys Leu Leu Asp Asp Thr Val Asp Thr Val Gln
       130                 135                 140
   Leu Gln Val Gly Ala Asn Ser Gly Glu Leu Ile Glu Leu Asp Leu Thr
   145                 150                 155                 160
   Lys Val Asp Leu Ser Ala Ile His Thr Ala Leu Ala Ala Glu Asp Ile
                   165                 170                 175
   Thr Asp His Thr Val Asp Glu Ser Gly Gly Asp Tyr Lys Asp Asp Asp
               180                 185                 190
   Asp Lys Gly Gly Val Asp Met Ile Leu Pro Trp Lys Trp Pro Trp Trp
           195                 200                 205
   Pro Trp Arg Arg Met Trp Ile Gln Asn Ala Gln Ser Ala Ile Asp Ala
       210                 215                 220
   Ile Asp Glu Gln Leu Lys Ala Val Ser Glu Gly Arg Ser Tyr Leu Gly
   225                 230                 235                 240
   Ala Met Gln Asn Arg Leu Glu His Thr Ile Lys Asn Leu Asp Asn Ala
                   245                 250                 255
   Ser Glu Asn Leu Gln Ala Ala Glu Ser Arg Ile Arg Asp Val Asp Met
               260                 265                 270
   Ala Lys Glu Met Met Glu Phe Thr Arg Thr Asn Ile Leu Asn Gln Ala
           275                 280                 285
   Ser Gln Ala Met Leu Ala Gln Ala Asn Gln Gln Pro Gln Ala Val Leu
       290                 295                 300
   Gln Leu Leu Arg
   305
<210> 47
   <211> 928
   <212> DNA
   <213> Bacillus halodurans
<220>
   <221> misc_feature
   <222> (541)..(649)
   <223> FliC nucleotide sequence incorporating the nucleotide sequence encoding the FLAG::Indolicidin (bp 541 - 649)
<400> 47
   atgattatca atcacaattt accagcaatg aatgcgcatc gtaacatggg tatcaatctt 60
   aaccaaggtc aagaagcgat ggagaagctt tcttcaggtc ttcgcattaa ccgtgcagga 120
   gacgatgctg caggtcttgc catctctgaa aaaatgcgtg cgcaaatccg tggtttggat 180
   caagcgtctc gtaactcaca agacggtatt tcgttaattc aaacagctga aggtgcgctt 240
   gatgaagtac attctattct tcaacgtatg cgtgagctag cggttcaatc ttcgaacgaa 300
   acgaatgttg agcaagatca agcagctctt aacgatgaat tccaacaatt agttgaggaa 360
   attgaaagaa tcaaagatac aactcaattt aatacgcaaa aattactcga tgatacagta 420
   gatactgtac aacttcaagt tggtgctaat tctggtgaat taattgaact tgatttaaca 480
   aaagttgatt tatcagctat ccatacagct ttggcggctg aggatattac tgaccacact 540
   gtcgacgaat ctggaggaga ttataaagat gatgatgata aaggaggagt cgacatgatc 600
   ctcgccgtgg aaatggccgt ggtggccgtg gcgtcgtatg tggatccaga atgcacaatc 660
   agctattgac gctattgatg agcaattaaa agctgtttca gaaggtcgct cttacctagg 720
   agctatgcaa aaccgcctag agcatacaat caaaaacctt gataatgctt ctgaaaacct 780
   tcaagctgct gagtctcgta tccgtgacgt agacatggcg aaagaaatga tggagttcac 840
   aagaacaaac atcttaaacc aagcgtctca agcgatgctt gctcaagcaa accaacagcc 900
   acaagctgta ttacaattac ttcgttaa 928
<210> 48
   <211> 331
   <212> PRT
   <213> Bacillus halodurans
<220>
   <221> MISC_FEATURE
   <222> (181)..(239)
   <223> FliC polypeptide sequence incorporating the FLAGx2::Fuzeon polypeptide sequence (aa 181 - 239)
<400> 48
   Met Ile Ile Asn His Asn Leu Pro Ala Met Asn Ala His Arg Asn Met
   1               5                   10                  15
   Gly Ile Asn Leu Asn Gln Gly Gln Glu Ala Met Glu Lys Leu Ser Ser
               20                  25                  30
   Gly Leu Arg Ile Asn Arg Ala Gly Asp Asp Ala Ala Gly Leu Ala Ile
           35                  40                  45
   Ser Glu Lys Met Arg Ala Gln Ile Arg Gly Leu Asp Gln Ala Ser Arg
       50                  55                  60
   Asn Ser Gln Asp Gly Ile Ser Leu Ile Gln Thr Ala Glu Gly Ala Leu
   65                  70                  75                  80
   Asp Glu Val His Ser Ile Leu Gln Arg Met Arg Glu Leu Ala Val Gln
                   85                  90                  95
   Ser Ser Asn Glu Thr Asn Val Glu Gln Asp Gln Ala Ala Leu Asn Asp
               100                 105                 110
   Glu Phe Gln Gln Leu Val Glu Glu Ile Glu Arg Ile Lys Asp Thr Thr
           115                 120                 125
   Gln Phe Asn Thr Gln Lys Leu Leu Asp Asp Thr Val Asp Thr Val Gln
       130                 135                 140
   Leu Gln Val Gly Ala Asn Ser Gly Glu Leu Ile Glu Leu Asp Leu Thr
   145                 150                 155                 160
   Lys Val Asp Leu Ser Ala Ile His Thr Ala Leu Ala Ala Glu Asp Ile
                   165                 170                 175
   Thr Asp His Thr Val Asp Glu Ser Gly Gly Asp Tyr Lys Asp Asp Asp
               180                 185                 190
   Asp Lys Gly Gly Val Asp Met Tyr Thr Ser Leu Ile His Ser Leu Ile
           195                 200                 205
   Glu Glu Ser Gln Asn Gln Gln Glu Lys Asn Glu Gln Glu Leu Leu Glu
       210                 215                 220
   Leu Asp Lys Trp Ala Ser Leu Trp Asn Trp Phe Met Trp Ile Gln Asn
   225                 230                 235                 240
   Ala Gln Ser Ala Ile Asp Ala Ile Asp Glu Gln Leu Lys Ala Val Ser
                   245                 250                 255
   Glu Gly Arg Ser Tyr Leu Gly Ala Met Gln Asn Arg Leu Glu His Thr
               260                 265                 270
   Ile Lys Asn Leu Asp Asn Ala Ser Glu Asn Leu Gln Ala Ala Glu Ser
           275                 280                 285
   Arg Ile Arg Asp Val Asp Met Ala Lys Glu Met Met Glu Phe Thr Arg
       290                 295                 300
   Thr Asn Ile Leu Asn Gln Ala Ser Gln Ala Met Leu Ala Gln Ala Asn
   305                 310                 315                 320
   Gln Gln Pro Gln Ala Val Leu Gln Leu Leu Arg
                   325                 330
<210> 49
   <211> 995
   <212> DNA
   <213> Bacillus halodurans
<220>
   <221> misc_feature
   <222> (541)..(717)
   <223> FliC nucleotide sequence incorporating the nucleotide sequence encoding FLAGx2::Fuzeon (bp 541 - 717)
<400> 49
   atgattatca atcacaattt accagcaatg aatgcgcatc gtaacatggg tatcaatctt     60
   aaccaaggtc aagaagcgat ggagaagctt tcttcaggtc ttcgcattaa ccgtgcagga    120
   gacgatgctg caggtcttgc catctctgaa aaaatgcgtg cgcaaatccg tggtttggat    180
   caagcgtctc gtaactcaca agacggtatt tcgttaattc aaacagctga aggtgcgctt    240
   gatgaagtac attctattct tcaacgtatg cgtgagctag cggttcaatc ttcgaacgaa    300
   acgaatgttg agcaagatca agcagctctt aacgatgaat tccaacaatt agttgaggaa    360
   attgaaagaa tcaaagatac aactcaattt aatacgcaaa aattactcga tgatacagta    420
   gatactgtac aacttcaagt tggtgctaat tctggtgaat taattgaact tgatttaaca    480
   aaagttgatt tatcagctat ccatacagct ttggcggctg aggatattac tgaccacact    540
   gtcgacgaat ctggaggaga ttataaagat gatgatgata aaggaggagt cgacatgtat    600
   acgtcattaa ttcattcatt aattgaagaa tcacaaaatc aacaagaaaa aaatgaacaa    660
   gaattattag aattagataa atgggcatca ttatggaatt ggtttatgtg gatccagatg    720
   cacaatcagc tattgacgct attgatgagc aattaaaagc tgtttcagaa ggtcgctctt    780
   acctaggagc tatgcaaaac cgcctagagc atacaatcaa aaaccttgat aatgcttctg    840
   aaaaccttca agctgctgag tctcgtatcc gtgacgtaga catggcgaaa gaaatgatgg    900
   agttcacaag aacaaacatc ttaaaccaag cgtctcaagc gatgcttgct caagcaaacc    960
   aacagccaca agctgtatta caattacttc gttaa                              995
<210> 50
   <211> 347
   <212> PRT
   <213> Bacillus halodurans
<220>
   <221> MISC_FEATURE
   <222> (181)..(254)
   <223> FliC polypeptide sequence incorporating the FLAGx2::Sifuvirtide polypeptide sequence (aa 181 - 254)
<400> 50
   Met Ile Ile Asn His Asn Leu Pro Ala Met Asn Ala His Arg Asn Met
   1               5                   10                  15
   Gly Ile Asn Leu Asn Gln Gly Gln Glu Ala Met Glu Lys Leu Ser Ser
   20 25 30
   Gly Leu Arg Ile Asn Arg Ala Gly Asp Asp Ala Ala Gly Leu Ala Ile
   35 40 45
   Ser Glu Lys Met Arg Ala Gln Ile Arg Gly Leu Asp Gln Ala Ser Arg
   50 55 60
   Asn Ser Gln Asp Gly Ile Ser Leu Ile Gln Thr Ala Glu Gly Ala Leu
   65 70 75 80
   Asp Glu Val His Ser Ile Leu Gln Arg Met Arg Glu Leu Ala Val Gln
   85 90 95
   Ser Ser Asn Glu Thr Asn Val Glu Gln Asp Gln Ala Ala Leu Asn Asp
   100 105 110
   Glu Phe Gln Gln Leu Val Glu Glu Ile Glu Arg Ile Lys Asp Thr Thr
   115 120 125
   Gln Phe Asn Thr Gln Lys Leu Leu Asp Asp Thr Val Asp Thr Val Gln
   130 135 140
   Leu Gln Val Gly Ala Asn Ser Gly Glu Leu Ile Glu Leu Asp Leu Thr
   145 150 155 160
   Lys Val Asp Leu Ser Ala Ile His Thr Ala Leu Ala Ala Glu Asp Ile
   165 170 175
   Thr Asp His Thr Val Asp Glu Ser Gly Gly Asp Tyr Lys Asp Asp Asp
   180 185 190
   Asp Lys Gly Gly Val Asp Glu Ser Gly Gly Asp Tyr Lys Asp Asp Asp
   195 200 205
   Asp Lys Gly Gly Val Asp Met Ser Trp Glu Thr Trp Glu Arg Glu Ile
   210 215 220
   Glu Asn Tyr Thr Arg Gln Ile Tyr Arg Ile Leu Glu Glu Ser Gln Glu
   225 230 235 240
   Gln Gln Asp Arg Asn Glu Arg Asp Leu Leu Glu Met Trp Ile Gln Asn
   245 250 255
   Ala Gln Ser Ala Ile Asp Ala Ile Asp Glu Gln Leu Lys Ala Val Ser
   260 265 270
   Glu Gly Arg Ser Tyr Leu Gly Ala Met Gln Asn Arg Leu Glu His Thr
   275 280 285
   Ile Lys Asn Leu Asp Asn Ala Ser Glu Asn Leu Gln Ala Ala Glu Ser
   290 295 300
   Arg Ile Arg Asp Val Asp Met Ala Lys Glu Met Met Glu Phe Thr Arg
   305 310 315 320
   Thr Asn Ile Leu Asn Gln Ala Ser Gln Ala Met Leu Ala Gln Ala Asn
   325 330 335
   Gln Gln Pro Gln Ala Val Leu Gln Leu Leu Arg
   340 345
<210> 51
   <211> 983
   <212> DNA
   <213> Bacillus halodurans
<220>
   <221> misc feature
   <222> (541)..(706)
   <223> FliC nucleotide sequence incorporating the nucleotide sequence encoding FLAGx2::Sifuvirtide (bp 541 - 706)
<400> 51
   atgattatca atcacaattt accagcaatg aatgcgcatc gtaacatggg tatcaatctt 60
   aaccaaggtc aagaagcgat ggagaagctt tcttcaggtc ttcgcattaa ccgtgcagga 120
   gacgatgctg caggtcttgc catctctgaa aaaatgcgtg cgcaaatccg tggtttggat 180
   caagcgtctc gtaactcaca agacggtatt tcgttaattc aaacagctga aggtgcgctt 240
   gatgaagtac attctattct tcaacgtatg cgtgagctag cggttcaatc ttcgaacgaa 300
   acgaatgttg agcaagatca agcagctctt aacgatgaat tccaacaatt agttgaggaa 360
   attgaaagaa tcaaagatac aactcaattt aatacgcaaa aattactcga tgatacagta 420
   gatactgtac aacttcaagt tggtgctaat tctggtgaat taattgaact tgatttaaca 480
   aaagttgatt tatcagctat ccatacagct ttggcggctg aggatattac tgaccacact 540
   gtcgacgaat ctggaggaga ttataaagat gatgatgata aaggaggagt ggaaacgtgg 600
   gaacgtgaaa tcgaaaatta tacgcgtcaa atctatcgta tcttagaaga atcacaagaa 660
   caacaagatc gtaatgaacg tgatttatta gaaatgtgga tccagaatgc acaatcagct 720
   attgacgcta ttgatgagca attaaaagct gtttcagaag gtcgctctta cctaggagct 780
   atgcaaaacc gcctagagca tacaatcaaa aaccttgata atgcttctga aaaccttcaa 840
   gctgctgagt ctcgtatccg tgacgtagac atggcgaaag aaatgatgga gttcacaaga 900
   acaaacatct taaaccaagc gtctcaagcg atgcttgctc aagcaaacca acagccacaa 960
   gctgtattac aattacttcg taa 983
<210> 52
   <211> 24
   <212> PRT
   <213> Artificial Sequence
<220>
   <223> HIV-antigenic peptide
<400> 52
   Thr Arg Pro Asn Asn Asn Thr Arg Lys Ser Ile Arg Ile Gly Pro Gly
   1               5                   10                  15
   Gln Thr Phe Tyr Ala Thr Gly Asp
               20
<210> 53
   <211> 27
   <212> PRT
   <213> Artificial Sequence
<220>
   <223> FLAG-Indolicidin
<400> 53
   Asp Tyr Lys Asp Asp Asp Asp Lys Gly Gly Val Asp Met Ile Leu Pro
   1               5                   10                  15
   Trp Lys Trp Pro Trp Trp Pro Trp Arg Arg Met
               20                  25
<210> 54
   <211> 50
   <212> PRT
   <213> Artificial Sequence
<220>
   <223> FLAG-Fuzeon
<400> 54
   Asp Tyr Lys Asp Asp Asp Asp Lys Gly Gly Val Asp Met Tyr Thr Ser
   1 5 10 15
   Leu Ile His Ser Leu Ile Glu Glu Ser Gln Asn Gln Gln Glu Lys Asn
   20 25 30
   Glu Gln Glu Leu Leu Glu Leu Asp Lys Trp Ala Ser Leu Trp Asn Trp
   35 40 45
   Phe Met
   50
<210> 55
   <211> 66
   <212> PRT
   <213> Artificial Sequence
<220>
   <223> FLAGx2-Sifuvirtide
<400> 55
   Asp Tyr Lys Asp Asp Asp Asp Lys Gly Gly Val Asp Glu Ser Gly Gly
   1 5 10 15
   Asp Tyr Lys Asp Asp Asp Asp Lys Gly Gly Val Asp Met Ser Trp Glu
   20 25 30
   Thr Trp Glu Arg Glu Ile Glu Asn Tyr Thr Arg Gln Ile Tyr Arg Ile
   35 40 45
   Leu Glu Glu Ser Gln Glu Gln Gln Asp Arg Asn Glu Arg Asp Leu Leu
   50 55 60
   Glu Met
   65
<210> 56
   <211> 65
   <212> PRT
   <213> Artificial Sequence
<220>
   <223> FLAGx2- Sifuveirtide ligated to the the N-terminal
<400> 56
   Asp Tyr Lys Asp Asp Asp Asp Lys Gly Gly Val Asp Glu Ser Gly Gly
   1               5                   10                  15
   Asp Tyr Lys Asp Asp Asp Asp Lys Gly Gly Val Asp Met Ser Trp Glu
               20                  25                  30
   Thr Trp Glu Arg Glu Ile Glu Asn Tyr Thr Arg Gln Ile Tyr Arg Ile
           35                  40                  45
   Leu Glu Glu Ser Gln Glu Gln Gln Asp Arg Asn Glu Arg Asp Leu Leu
       50                  55                  60
   Glu
   65
<210> 57
   <211> 49
   <212> PRT
   <213> Artificial Sequence
<220>
   <223> FLAG-Sifuvirtide ligated to the C-terminal
<400> 57
   Asp Tyr Lys Asp Asp Asp Asp Lys Gly Gly Val Asp Met Ser Trp Glu
   1               5                   10                  15
   Thr Trp Glu Arg Glu Ile Glu Asn Tyr Thr Arg Gln Ile Tyr Arg Ile
               20                  25                  30
   Leu Glu Glu Ser Gln Glu Gln Gln Asp Arg Asn Glu Arg Asp Leu Leu
           35                  40                  45
   Glu
<210> 58
   <211> 93
   <212> DNA
   <213> Artificial Sequence
<220>
   <223> Complementary forward oligonucleotide sequence used in the synthesis of the HIV-antigenic peptide
<400> 58
   ggctgtcgac acgcgtccaa ataataatac gcgtaaatca attcgtattg gaccaggaca 60
   aacgttttat gcaacgggag attggatcca ggg 93
<210> 59
   <211> 93
   <212> DNA
   <213> Artificial Sequence
<220>
   <223> Complementary reverse oligonucleotide sequence used in the synthesis of the HIV-antigenic peptide
<400> 59
   ccgcggatcc aatctcccgt tgcataaaac gtttgtcctg gtccaatacg aattgattta 60
   cgcgtattat tatttggacg cgtgtcgacg ccc 93
<210> 60
   <211> 64
   <212> DNA
   <213> Artificial Sequence
<220>
   <223> Complementary forward oligonucleotide sequence used in the synthesis of Indolicidin
<400> 60
   caggtcgaca tgatcctgcc gtggaaatgg ccgtggtggc cgtggcgtcg tatgtggatc 60
   cagc 64
<210> 61
   <211> 64
   <212> DNA
   <213> Artificial Sequence
<220>
   <223> Complementary reverse oligonucleotide sequence used in the synthesis of Indolicidin
<400> 61
   gctggatcca catacgacgc cacggccacc acggccattt ccacggcagg atcatgtcga 60
   cctg 64
<210> 62
   <211> 133
   <212> DNA
   <213> Artificial Sequence
<220>
   <223> Complementary forward oligonucleotide sequence used in the synthesis of Fuzeon
<400> 62
   gctgtcgaca tgtatacgtc attaattcat tcattaattg aagaatcaca aaatcaacaa 60
   gaaaaaaatg aacaagaatt attagaatta gataaatggg catcattatg gaattggttt 120
   atgtggatcc agg 133
<210> 63
   <211> 133
   <212> DNA
   <213> Artificial Sequence
<220>
   <223> Complementary reverse oligonucleotide sequence used in the synthesis of Fuzeon
<400> 63
   cctggatcca cataaaccaa ttccataatg atgcccattt atctaattct aataattctt     60
   gttcattttt ttcttgttga ttttgtgatt cttcaattaa agtatgaatt aatgacgtat    120
   acatgtcgac agc                                                        133
<210> 64
   <211> 138
   <212> DNA
   <213> Artificial Sequence
<220>
   <223> Complementary forward oligonucleotide sequence used in the synthesis of Sifuvirtide
<400> 64
   gcagctgtcg acatgtcatg ggaaacgtgg gaacgtgaaa tcgaaaatta tacgcgtcaa     60
   atctatcgta tcttagaaga atcacaagaa caacaagatc gtaatgaacg tgatttatta    120
   gaaatgtgga tccaggtc                                                   138
<210> 65
   <211> 138
   <212> DNA
   <213> Artificial Sequence
<220>
   <223> Complementary reverse oligonucleotide sequence used in the synthesis of Sifuvirtide
<400> 65
   gacctggatc cacatttcta ataaatcacg ttcattacga tcttgttgtt cttgtgattc     60
   ttctaagata cgatagattt gacgcgtata attttcgatt tcacgttccc acgtttccca    120
   tgacatgtcg acagctgc                                                   138
<210> 66
   <211> 48
   <212> DNA
   <213> Artificial Sequence
<220>
   <223> Complementary forward oligonucleotide sequence used in the synthesis of the FLAG-tag
<400> 66
   tcgacgaatc tggaggagat tataaagatg atgatgataa aggaggag 48
<210> 67
   <211> 48
   <212> DNA
   <213> Artificial Sequence
<220>
   <223> Complementary reverse oligonucleotide sequence used in the synthesis of the FLAG-tag
<400> 67
   tcgactcctc ctttatcatc atcatcttta taatctcctc cagattcg 48
<210> 68
   <211> 251
   <212> PRT
   <213> Bacillus halodurans
<220>
   <221> MISC_FEATURE
   <222> (181)..(251)
   <223> FliC N-terminal fragment polypeptide sequence fused to the FLAG::Sifuvirtide polypeptide sequence (aa 181 - 251)
<400> 68
   Met Ile Ile Asn His Asn Leu Pro Ala Met Asn Ala His Arg Asn Met
   1               5                   10                  15
   Gly Ile Asn Leu Asn Gln Gly Gln Glu Ala Met Glu Lys Leu Ser Ser
               20                  25                  30
   Gly Leu Arg Ile Asn Arg Ala Gly Asp Asp Ala Ala Gly Leu Ala Ile
           35                  40                  45
   Ser Glu Lys Met Arg Ala Gln Ile Arg Gly Leu Asp Gln Ala Ser Arg
       50                  55                  60
   Asn Ser Gln Asp Gly Ile Ser Leu Ile Gln Thr Ala Glu Gly Ala Leu
   65                  70                  75                  80
   Asp Glu Val His Ser Ile Leu Gln Arg Met Arg Glu Leu Ala Val Gln
                   85                  90                  95
   Ser Ser Asn Glu Thr Asn Val Glu Gln Asp Gln Ala Ala Leu Asn Asp
               100                 105                 110
   Glu Phe Gln Gln Leu Val Glu Glu Ile Glu Arg Ile Lys Asp Thr Thr
           115                 120                 125
   Gln Phe Asn Thr Gln Lys Leu Leu Asp Asp Thr Val Asp Thr Val Gln
       130                 135                 140
   Leu Gln Val Gly Ala Asn Ser Gly Glu Leu Ile Glu Leu Asp Leu Thr
   145                 150                 155                 160
   Lys Val Asp Leu Ser Ala Ile His Thr Ala Leu Ala Ala Glu Asp Ile
                   165                 170                 175
   Thr Asp His Thr Val Asp Glu Ser Gly Gly Asp Tyr Lys Asp Asp Asp
               180                 185                 190
   Asp Lys Gly Gly Val Asp Glu Ser Gly Gly Asp Tyr Lys Asp Asp Asp
           195                 200                 205
   Asp Lys Gly Gly Val Asp Met Ser Trp Glu Thr Trp Glu Arg Glu Ile
       210                 215                 220
   Glu Asn Tyr Thr Arg Gln Ile Tyr Arg Ile Leu Glu Glu Ser Gln Glu
   225                 230                 235                 240
   Gln Gln Asp Arg Asn Glu Arg Asp Leu Leu Glu
                   245                 250
<210> 69
   <211> 756
   <212> DNA
   <213> Bacillus halodurans
<220>
   <221> misc_feature
   <222> (541)..(756)
   <223> FliC N-terminal fragment nucleotide sequence incorporating the nucleotide sequence encoding FLAG::Sifuvirtide (bp 541 - 756)
<400> 69
   atgattatca atcacaattt accagcaatg aatgcgcatc gtaacatggg tatcaatctt 60
   aaccaaggtc aagaagcgat ggagaagctt tcttcaggtc ttcgcattaa ccgtgcagga 120
   gacgatgctg caggtcttgc catctctgaa aaaatgcgtg cgcaaatccg tggtttggat 180
   caagcgtctc gtaactcaca agacggtatt tcgttaattc aaacagctga aggtgcgctt 240
   gatgaagtac attctattct tcaacgtatg cgtgagctag cggttcaatc ttcgaacgaa 300
   acgaatgttg agcaagatca agcagctctt aacgatgaat tccaacaatt agttgaggaa 360
   attgaaagaa tcaaagatac aactcaattt aatacgcaaa aattactcga tgatacagta 420
   gatactgtac aacttcaagt tggtgctaat tctggtgaat taattgaact tgatttaaca 480
   aaagttgatt tatcagctat ccatacagct ttggcggctg aggatattac tgaccacact 540
   gtcgacgaat ctggaggaga ttataaagat gatgatgata aaggaggagt cgacgaatct 600
   ggaggagatt ataaagatga tgatgataaa ggaggagtcg acatgtcatg ggaaacgtgg 660
   gaacgtgaaa tcgaaaatta tacgcgtcaa atctatcgta tcttagaaga atcacaagaa 720
   caacaagatc gtaatgaacg tgatttatta gaataa 756
<210> 70
   <211> 327
   <212> PRT
   <213> Bacillus halodurans
<220>
   <221> MISC_FEATURE
   <222> (273)..(327)
   <223> FliC polypeptide sequence fused to the polypeptide sequence of FLAGx2::Sifuvirtide polypeptide sequence (aa 273 - 327)
<400> 70
   Met Ile Ile Asn His Asn Leu Pro Ala Met Asn Ala His Arg Asn Met
   1               5                   10                  15
   Gly Ile Asn Leu Asn Gln Gly Gln Glu Ala Met Glu Lys Leu Ser Ser
               20                  25                  30
   Gly Leu Arg Ile Asn Arg Ala Gly Asp Asp Ala Ala Gly Leu Ala Ile
           35                  40                  45
   Ser Glu Lys Met Arg Ala Gln Ile Arg Gly Leu Asp Gln Ala Ser Arg
       50                  55                  60
   Asn Ser Gln Asp Gly Ile Ser Leu Ile Gln Thr Ala Glu Gly Ala Leu
   65                  70                  75                  80
   Asp Glu Val His Ser Ile Leu Gln Arg Met Arg Glu Leu Ala Val Gln
                   85                  90                  95
   Ser Ser Asn Glu Thr Asn Val Glu Gln Asp Gln Ala Ala Leu Asn Asp
               100                 105                 110
   Glu Phe Gln Gln Leu Val Glu Glu Ile Glu Arg Ile Lys Asp Thr Thr
           115                 120                 125
   Gln Phe Asn Thr Gln Lys Leu Leu Asp Asp Thr Val Asp Thr Val Gln
       130                 135                 140
   Leu Gln Val Gly Ala Asn Ser Gly Glu Leu Ile Glu Leu Asp Leu Thr
   145                 150                 155                 160
   Lys Val Asp Leu Ser Ala Ile His Thr Ala Leu Ala Ala Glu Asp Ile
                   165                 170                 175
   Thr Asp His Thr Asn Ala Gln Ser Ala Ile Asp Ala Ile Asp Glu Gln
               180                 185                 190
   Leu Lys Ala Val Ser Glu Gly Arg Ser Tyr Leu Gly Ala Met Gln Asn
           195                 200                 205
   Arg Leu Glu His Thr Ile Lys Asn Leu Asp Asn Ala Ser Glu Asn Leu
       210                 215                 220
   Gln Ala Ala Glu Ser Arg Ile Arg Asp Val Asp Met Ala Lys Glu Met
   225                 230                 235                 240
   Met Glu Phe Thr Arg Thr Asn Ile Leu Asn Gln Ala Ser Gln Ala Met
                   245                 250                 255
   Leu Ala Gln Ala Asn Gln Gln Pro Gln Ala Val Leu Gln Leu Leu Arg
   260 265 270
   Leu Asp Glu Ser Gly Ala Asp Tyr Lys Asp Asp Asp Asp Lys Gly Gly
   275 280 285
   Val Asp Met Ser Trp Glu Thr Trp Glu Arg Glu Ile Glu Asn Tyr Thr
   290 295 300
   Arg Gln Ile Tyr Arg Ile Ile Glu Glu Ser Gln Glu Gln Gln Asp Arg
   305 310 315 320
   Asn Glu Arg Asp Leu Leu Glu
   325
<210> 71
   <211> 984
   <212> PRT
   <213> Bacillus halodurans
<220>
   <221> MISC_FEATURE
   <222> (817)..(984)
   <223> FiC nucleotide sequence incorporating the nucleotide sequence encoding FLAGx2::Sifuvirtide (bp 817 - 984)
<400> 71
   Ala Thr Gly Ala Thr Thr Ala Thr Cys Ala Ala Thr Cys Ala Cys Ala
   1 5 10 15
   Ala Thr Thr Thr Ala Cys Cys Ala Gly Cys Ala Ala Thr Gly Ala Ala
   20 25 30
   Thr Gly Cys Gly Cys Ala Thr Cys Gly Thr Ala Ala Cys Ala Thr Gly
   35 40 45
   Gly Gly Thr Ala Thr Cys Ala Ala Thr Cys Thr Thr Ala Ala Cys Cys
   50 55 60
   Ala Ala Gly Gly Thr Cys Ala Ala Gly Ala Ala Gly Cys Gly Ala Thr
   65 70 75 80
   Gly Gly Ala Gly Ala Ala Gly Cys Thr Thr Thr Cys Thr Thr Cys Ala
   85 90 95
   Gly Gly Thr Cys Thr Thr Cys Gly Cys Ala Thr Thr Ala Ala Cys Cys
   100 105 110
   Gly Thr Gly Cys Ala Gly Gly Ala Gly Ala Cys Gly Ala Thr Gly Cys
   115 120 125
   Thr Gly Cys Ala Gly Gly Thr Cys Thr Thr Gly Cys Cys Ala Thr Cys
   130 135 140
   Thr Cys Thr Gly Ala Ala Ala Ala Ala Ala Thr Gly Cys Gly Thr Gly
   145 150 155 160
   Cys Gly Cys Ala Ala Ala Thr Cys Cys Gly Thr Gly Gly Thr Thr Thr
   165 170 175
   Gly Gly Ala Thr Cys Ala Ala Gly Cys Gly Thr Cys Thr Cys Gly Thr
   180 185 190
   Ala Ala Cys Thr Cys Ala Cys Ala Ala Gly Ala Cys Gly Gly Thr Ala
   195 200 205
   Thr Thr Thr Cys Gly Thr Thr Ala Ala Thr Thr Cys Ala Ala Ala Cys
   210 215 220
   Ala Gly Cys Thr Gly Ala Ala Gly Gly Thr Gly Cys Gly Cys Thr Thr
   225 230 235 240
   Gly Ala Thr Gly Ala Ala Gly Thr Ala Cys Ala Thr Thr Cys Thr Ala
   245 250 255
   Thr Thr Cys Thr Thr Cys Ala Ala Cys Gly Thr Ala Thr Gly Cys Gly
   260 265 270
   Thr Gly Ala Gly Cys Thr Ala Gly Cys Gly Gly Thr Thr Cys Ala Ala
   275 280 285
   Thr Cys Thr Thr Cys Gly Ala Ala Cys Gly Ala Ala Ala Cys Gly Ala
   290 295 300
   Ala Thr Gly Thr Thr Gly Ala Gly Cys Ala Ala Gly Ala Thr Cys Ala
   305 310 315 320
   Ala Gly Cys Ala Gly Cys Thr Cys Thr Thr Ala Ala Cys Gly Ala Thr
   325 330 335
   Gly Ala Ala Thr Thr Cys Cys Ala Ala Cys Ala Ala Thr Thr Ala Gly
   340 345 350
   Thr Thr Gly Ala Gly Gly Ala Ala Ala Thr Thr Gly Ala Ala Ala Gly
   355 360 365
   Ala Ala Thr Cys Ala Ala Ala Gly Ala Thr Ala Cys Ala Ala Cys Thr
       370                 375                 380
   Cys Ala Ala Thr Thr Thr Ala Ala Thr Ala Cys Gly Cys Ala Ala Ala
   385                 390                 395                 400
   Ala Ala Thr Thr Ala Cys Thr Cys Gly Ala Thr Gly Ala Thr Ala Cys
                   405                 410                 415
   Ala Gly Thr Ala Gly Ala Thr Ala Cys Thr Gly Thr Ala Cys Ala Ala
               420                 425                 430
   Cys Thr Thr Cys Ala Ala Gly Thr Thr Gly Gly Thr Gly Cys Thr Ala
           435                 440                 445
   Ala Thr Thr Cys Thr Gly Gly Thr Gly Ala Ala Thr Thr Ala Ala Thr
       450                 455                 460
   Thr Gly Ala Ala Cys Thr Thr Gly Ala Thr Thr Thr Ala Ala Cys Ala
   465                 470                 475                 480
   Ala Ala Ala Gly Thr Thr Gly Ala Thr Thr Thr Ala Thr Cys Ala Gly
                   485                 490                 495
   Cys Thr Ala Thr Cys Cys Ala Thr Ala Cys Ala Gly Cys Thr Thr Thr
               500                 505                 510
   Gly Gly Cys Gly Gly Cys Thr Gly Ala Gly Gly Ala Thr Ala Thr Thr
           515                 520                 525
   Ala Cys Thr Gly Ala Cys Cys Ala Cys Ala Cys Thr Ala Ala Thr Gly
       530                 535                 540
   Cys Ala Cys Ala Ala Thr Cys Ala Gly Cys Thr Ala Thr Thr Gly Ala
   545                 550                 555                 560
   Cys Gly Cys Thr Ala Thr Thr Gly Ala Thr Gly Ala Gly Cys Ala Ala
                   565                 570                 575
   Thr Thr Ala Ala Ala Ala Gly Cys Thr Gly Thr Thr Thr Cys Ala Gly
               580                 585                 590
   Ala Ala Gly Gly Thr Cys Gly Cys Thr Cys Thr Thr Ala Cys Cys Thr
           595                 600                 605
   Ala Gly Gly Ala Gly Cys Thr Ala Thr Gly Cys Ala Ala Ala Ala Cys
       610                 615                 620
   Cys Gly Cys Cys Thr Ala Gly Ala Gly Cys Ala Thr Ala Cys Ala Ala
   625 630 635 640
   Thr Cys Ala Ala Ala Ala Ala Cys Cys Thr Thr Gly Ala Thr Ala Ala
   645 650 655
   Thr Gly Cys Thr Thr Cys Thr Gly Ala Ala Ala Ala Cys Cys Thr Thr
   660 665 670
   Cys Ala Ala Gly Cys Thr Gly Cys Thr Gly Ala Gly Thr Cys Thr Cys
   675 680 685
   Gly Thr Ala Thr Cys Cys Gly Thr Gly Ala Cys Gly Thr Ala Gly Ala
   690 695 700
   Cys Ala Thr Gly Gly Cys Gly Ala Ala Ala Gly Ala Ala Ala Thr Gly
   705 710 715 720
   Ala Thr Gly Gly Ala Gly Thr Thr Cys Ala Cys Ala Ala Gly Ala Ala
   725 730 735
   Cys Ala Ala Ala Cys Ala Thr Cys Thr Thr Ala Ala Ala Cys Cys Ala
   740 745 750
   Ala Gly Cys Gly Thr Cys Thr Cys Ala Ala Gly Cys Gly Ala Thr Gly
   755 760 765
   Cys Thr Thr Gly Cys Thr Cys Ala Ala Gly Cys Ala Ala Ala Cys Cys
   770 775 780
   Ala Ala Cys Ala Gly Cys Cys Ala Cys Ala Ala Gly Cys Thr Gly Thr
   785 790 795 800
   Ala Thr Thr Ala Cys Ala Ala Thr Thr Ala Cys Thr Thr Cys Gly Thr
   805 810 815
   Cys Thr Cys Gly Ala Cys Gly Ala Ala Thr Cys Thr Gly Gly Ala Gly
   820 825 830
   Cys Ala Gly Ala Thr Thr Ala Thr Ala Ala Ala Gly Ala Thr Gly Ala
   835 840 845
   Thr Gly Ala Thr Gly Ala Thr Ala Ala Ala Gly Gly Ala Gly Gly Ala
   850 855 860
   Gly Thr Cys Gly Ala Cys Ala Thr Gly Thr Cys Ala Thr Gly Gly Gly
   865                 870                 875                 880
   Ala Ala Ala Cys Gly Thr Gly Gly Gly Ala Ala Cys Gly Thr Gly Ala
                   885                 890                 895
   Ala Ala Thr Cys Gly Ala Ala Ala Ala Thr Thr Ala Thr Ala Cys Gly
               900                 905                 910
   Cys Gly Thr Cys Ala Ala Ala Thr Cys Thr Ala Thr Cys Gly Thr Ala
           915                 920                 925
   Thr Cys Thr Thr Ala Gly Ala Ala Gly Ala Ala Thr Cys Ala Cys Ala
       930                 935                 940
   Ala Gly Ala Ala Cys Ala Ala Cys Ala Ala Gly Ala Thr Cys Gly Thr
   945                 950                 955                 960
   Ala Ala Thr Gly Ala Ala Cys Gly Thr Gly Ala Thr Thr Thr Ala Thr
                   965                 970                 975
   Thr Ala Gly Ala Ala Thr Ala Ala
               980

## Description

THIS INVENTION relates to the production of heterologous proteins or peptides by Gram-positive bacterial host cells.

### Background

PCT International application PCT/IB2005/054022 (International publication number WO 2006/072845) describes recombinant Gram-positive bacterial strain (*B. halodurans* Alk36) which has the ability to over-produce flagellin protein (FliC) when compared to other Gram-positive bacterial strains. The recombinant strain produces high levels of stable and soluble recombinant flagellin protein on the cell surface of the recombinant strain. In order to achieve this, the recombinant strain is genetically modified to facilitate the expression of a chimeric polypeptide comprising a flagellin monomer and a peptide of choice inserted into the central variable region thereof. The genetic modifications include (i) Inactivating the *hag* gene on the chromosome which codes for functional flagellin; (ii) inactivating the cell wall protease *wpr*A; and (iii) transforming the recombinant strain with a multicopy vector containing the gene encoding an in-frame flagellin peptide fusion protein.

### Summary of Invention

The inventors have developed a method for making therapeutic peptides utilizing a modified flagella type III secretion system whereby the therapeutic peptides are exported into the growth medium by a modified *B. halodurans* Alk36 strain (NCIMB 41533).

The modifications include inactivation of the flagellin gene (*hag* gene) by a disruption preventing expression of a functional flagellin. The disruption can be by replacement of an endogenous gene with a DNA sequence encoding either no polypeptide or a non-functional flagellin polypeptide. In this case, the non-functional flagellin polypeptide is a deletion mutant lacking amino acids 14 to 226 of SEQ ID NO: 1. The disruption of the *hag* gene is fully described in the PCT International application PCT/IB2005/054022 (International publication number WO 2006/072845), which is fully herein incorporated by reference.

Export of chimeric flagellin monomers was achieved by altering the genome of the *B. halodurans* (Δ*hag*) strain through targeted inactivation of a *fliD* gene encoding a flagellin cap protein in addition to the genetic modification disclosed in the PCT International application PCT/IB2005/054022 (International publication number WO 2006/072845). The cap protein aids polymerization of the flagellin monomers to form a flagellin filament. The cap protein comprises 5 FliD subunits located at the tip of the flagellin filament and needs to be in place for polymerization of flagellin protein to take place. Inactivation of the *fliD* gene results in secretion of un-polymerized chimeric flagellin monomers into the extracellular medium. In this case, the non-functional FliD polypeptide is of SEQ ID NO: 2.

Protease gene homologues to the key proteases as identified in *B. subtilis* from the literature were selected for gene targeted inactivation. The sequences were identified from a search of the *B*. *halodurans* C-125 genome as accessed from the DNA Data Bank of Japan (DDBJ; http://gib.genes.nig.ac.jp). These include *wprA* (BH2080), *alp* (BH0684; [SEQ ID NO. 3]*), vpr* (BH0831; [SEQ ID NO. 5],), *apr* (BH0696; [SEQ ID NO. 4]), asp (BH0855; [SEQ ID NO. 6]) and *aprX* (BH1930) genes

In order to improve the secretion ability of strain *B*. *halodurans* Alk36, its genome was further altered through targeted inactivation of these key protease genes. The resultant strain *B*. *halodurans* Alk36 (Δ*hag*, Δ*fliD*, Δ*wpr*A*,* Δ*alp*, Δ*apr*, Δ*vpr*, Δ*asp*), designated BhFD05, was transformed with an expression vector containing a fusion polypeptide linked to either the N-terminal or C-terminal flagellin region(s) or situated in a flagellin variable region, linked to both the N-terminal and C-terminal regions.

Thus, in accordance with a first aspect of the invention, there is provided a method of producing a flagellin-based chimeric protein, the method including
culturing a *B*. *halodurans* BhFD05 (Δ*hag*, Δ*fliD*, Δ*wprA*, Δ*alp*, Δ*apr*, Δ*vpr*, Δ*asp*) strain deposited under Accession Number 41533 at the NCIMB, and
causing the strain to express and secrete, into an extracellular growth medium, high levels of a flagellin-based chimeric protein comprising a heterologous peptide selected from Indolicidin, Enfuvirtide and Sifuvirtide, and (i) inserted in-frame into a flagellin variable region which is flanked on its N-terminal side by an N-terminal fragment of a flagellin polypeptide and, optionally, flanked on its C-terminal side by a C-terminal fragment of a flagellin polypeptide, or (ii) fused to the C-terminal of a flagellin polypeptide.

The N-terminal-, C-terminal- and variable regions of the *B halodurans* flagellin protein are as defined in PCT International application PCT/IB2005/054022 (International publication number WO 2006/072845).

*B. halodurans* BhFD05 was deposited under Accession Number NCIMB41533 on 17 December 2007 at NCIMB Ltd of Furguson Building, Craibstore Estate, Buchsburn, Aberdeen AB210YA.

The growth medium containing the chimeric protein may be usable as a crude preparation. The crude preparation may be a cell-free preparation.

The method may include partially or fully purifying the chimeric protein from the growth medium.

According to a second aspect of the invention, there is provided a flagellin-based chimeric protein produced by the method of the first aspect of the invention, and which comprises a heterologous peptide selected from Indolicidin, Enfuvirtide and Sifuvirtide, and (i) inserted in-frame into a flagellin variable region which is flanked on its N-terminal side by an N-terminal fragment of the flagellin polypeptide and, optionally, flanked on its C-terminal side by a C-terminal fragment of a flagellin polypeptide, or (ii) fused to the C-terminal of a flagellin polypeptide.

The heterologous peptide may be fused to only the N-terminal fragment of the flagellin polypeptide.

Instead, the heterologous peptide may be fused to the C-terminal of a full length flagellin polypeptide.

The flagellin-based chimeric protein may instead, or additionally, include a polypeptide tag fused to the N-terminal of the heterologous peptide. Such a tag may be used to isolate the chimeric protein. The tag may also be used as a specific target in Western blot analysis. The tag may be a known tag such as a FLAG-tag, a HIS-tag, or the like. More than one copy of the tag may also be fused to the N-terminal of the heterologous peptide.

The flagellin-based chimeric protein may instead, or additionally, include a cleavage site adjacent to at least one side of, or linked to, the heterologous region, ie the heterologous peptide. A cleavage site may be provided adjacent to both sides of the heterologous region, i.e. cleavage sites may flank the heterologous region. The cleavage site(s) may be known cleavage sites such as a methionine cleavage site which is recognised by chemical agents such as cyanogen bromide.

The heterologous peptide (or polypeptide) may be a therapeutic peptide, which may be selected from the group consisting of an antimicrobial peptide, an antiviral peptide and an immunogenic peptide.

'Enfuvirtide' is marketed as "Fuzeon" (trademark). "Sifuvirtide" is profiled as a promising improvement to Fuzeon.

The size of the heterologous peptides expressed ranged from 12- to 75 amino acids. Yields obtained after tag purification from the different constructs ranged from 2-20 mg/L.

The invention extends further to the use of the flagellin-based chimeric protein according to the second aspect of the invention, in the manufacture of a medicament for therapeutic use.

According to a third aspect of the invention, there is provided a nucleic acid encoding a chimeric protein according to the second aspect of the invention, the nucleic acid comprising a nucleotide sequence encoding (i) the N-terminal fragment of a flagellin polypeptide; the variable region of a flagellin polypeptide; optionally, the C-terminal fragment of a flagellin polypeptide, and a nucleotide sequence encoding a heterologous peptide selected from Indolicidin, Enfuvirtide and Sifuvirtide, and inserted in-frame into the nucleotide sequence encoding the variable region of the flagellin polypeptide, or (ii) a heterologous polypeptide selected from Indolicidin, Enfuvirtide and Sifuvirtide, and fused to the C-terminal of a flagellin polypeptide.

The nucleic acid may include a nucleotide sequence encoding the N-terminal fragment of the flagellin polypeptide ligated on its C-terminal end in-frame to a nucleotide sequence encoding a heterologous peptide.

The nucleotide sequence encoding the heterologous peptide may be inserted immediately after any nucleotide between nucleotide 162 and nucleotide 606 of SEQ ID NO: 7.

The nucleotide sequence encoding the heterologous peptide may be inserted as an in-frame fusion immediately after nucleotide 816 of SEQ ID NO: 7.

According to a fourth aspect of the invention, there is provided an expression cassette which includes a nucleic acid sequence encoding the chimeric protein according to the second aspect of the invention.

The nucleic acid sequence encoding the chimeric protein may be integrated into the chromosome of the host cell.

According to a fifth aspect of the invention, there is provided a nucleic acid vector which includes a nucleic acid sequence encoding the chimeric protein of the second aspect of the invention, operably linked to a transcriptional regulatory element (TRE).

The nucleic acid vector may be an extra-chromosomal plasmid.

According to a sixth aspect of the invention, there is provided a bacterial cell containing the nucleic acid vector of the fifth aspect of the invention.

The bacterial cell may be a Gram-positive bacterial cell such as a cell of the *Bacillus* genus, e.g., a cell of the *B. halodurans* species. The cell may be of the strain *B. halodurans* BhFD05 (Δ*hag*, Δ*fliD*, Δ*wpr*A, Δ*alp*, Δ*apr*, Δ*vpr*, Δ*asp*) deposited under Accession Number 41533 at the NCIMB.

The terms "polypeptide" and "protein" are used interchangeably to mean any peptide-linked chain of amino acids, regardless of length or post-translational modification.

Unless otherwise defined, all technical and scientific terms used herein have the same meaning as commonly understood by one of ordinary skill persons in the art to which this invention pertains. In case of conflict, the present document, including definitions, control. Preferred methods and materials are described below, although methods and materials similar or equivalent to those described herein can be used in the practice or testing of the present invention. All publications, patent applications, patents and other references mentioned herein are incorporated by reference in their entirety. The materials, methods, and examples disclosed herein are illustrative only and not intended to be limiting.

Further features of the invention will now be described with reference to the following non-limiting examples, sequence listings and accompanying drawings.

In the drawings,

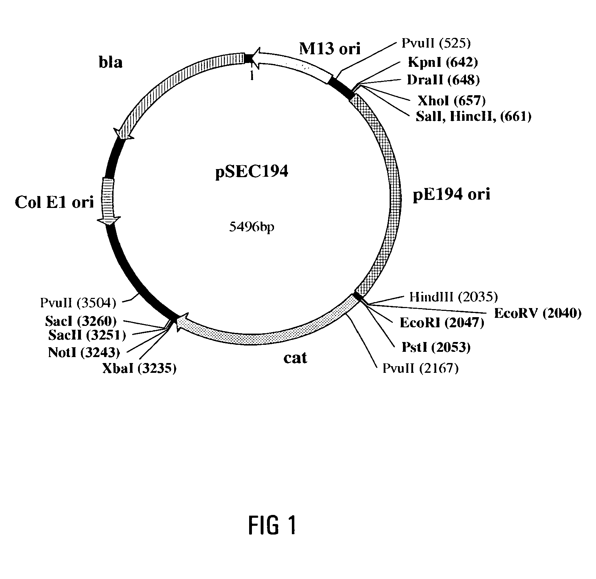

FIGURE 1: Plasmid map of pSEC194 (5.496 kb). Temperature sensitive ori (pE194) and ColE1 ori used for replication in *Bacillus* and *E*. *coli* respectively. Restriction enzyme sites (bold), useful for cloning. Plasmid map created with DNAMAN, Version 4.1;

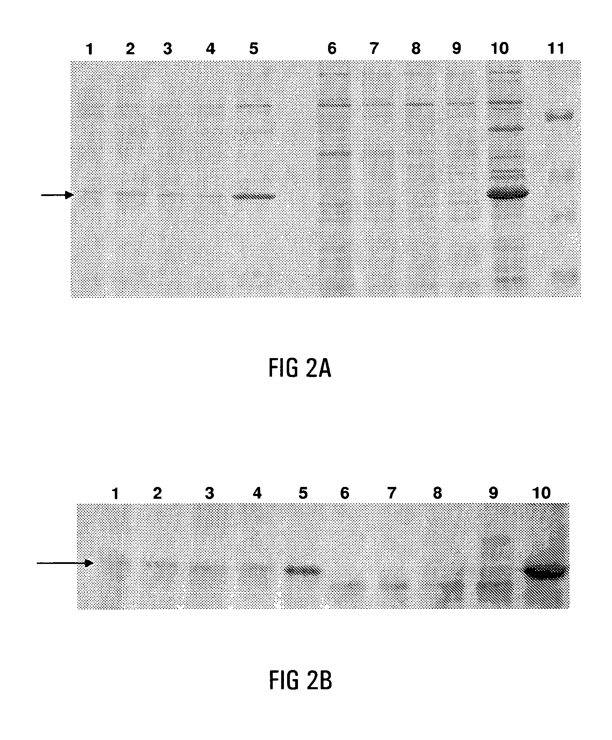

FIGURE 2A: SDS-PAGE comparison of extracellular samples from different protease deficient *B. halodurans* strains secreting HIV antigenic fusion peptide at pH 8.5 during exponential (lanes 1-5, OD₆₀₀ 1.6) and stationary (lanes 6-10, OD₆₀₀ 5) phase. Lanes 1 and 6, strain BhFD01, lanes 2 and 7, strain BhFD02; lanes 3 and 8, BhFD03; lanes 4 and 9, BhFD04; lanes 5 and 10, BhFD05 and lane 11, molecular weight marker (Fermentas). The arrow indicates the HIV antigenic fusion peptide;
FIGURE 2B. Western blot using anti-FLAG antibodies against the chimeric flagellin carrying the HIV antigenic peptide. The arrow indicates the FLAG HIV antigenic fusion peptide;

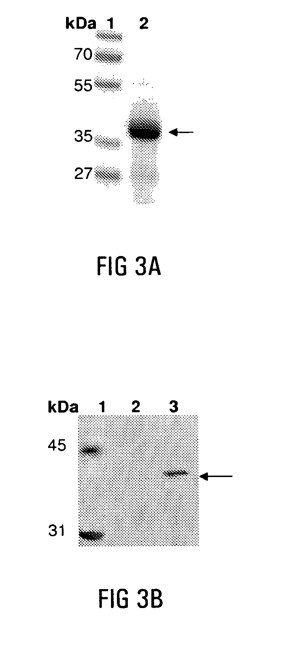

FIGURE 3A: SDS PAGE gel analysis of the extracellular protein fraction of chimeric flagellin in *B. halodurans* strain BhFD05 after chromatography. Lane 1, molecular weight marker (Fermentas); lane 2, pSECNHIVC7 FLAG-affinity eluate. The arrow indicates the flagellin-HIV antigenic fusion protein;
FIGURE 3B: Western blot analysis of 3A using MEIV3b 4 antibodies. Lane 1, low molecular mass marker (Biorad); lane 2, pSECNC7 (negative control) and lane 3, pSECNHIVC7. The arrow indicates the flagellin-HIV antigenic fusion protein;

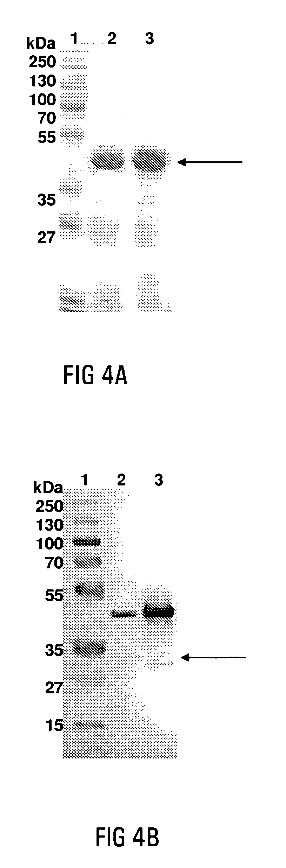

FIGURE 4A: SDS PAGE gel analysis of the extra-cellular protein fraction of chimeric flagellin carrying the FLAG-tag in *B*. *halodurans* strain BhFD05 after FLAG-affinity chromatography. Lane 1, low molecular mass ladder (Fermentas); lane 2, pSECNFFuzC7 and lane 3, pSECNF2SifC7. The arrow indicates Fuzeon^{™} and Sifuvirtide flagellin fusion proteins;
FIGURE 4B: Western blot analysis using anti-FLAG antibodies against the extracellular protein fractions from the chimeric flagellin expression cassettes in *B. halodurans* BhFD05. Lane 1, low molecular mass marker; lane 2, pSECNF2SifC7; and lane 3, pSECNFFuzC7. The arrow indicates Fuzeon^{™} and Sifuvirtide flagellin fusion proteins;

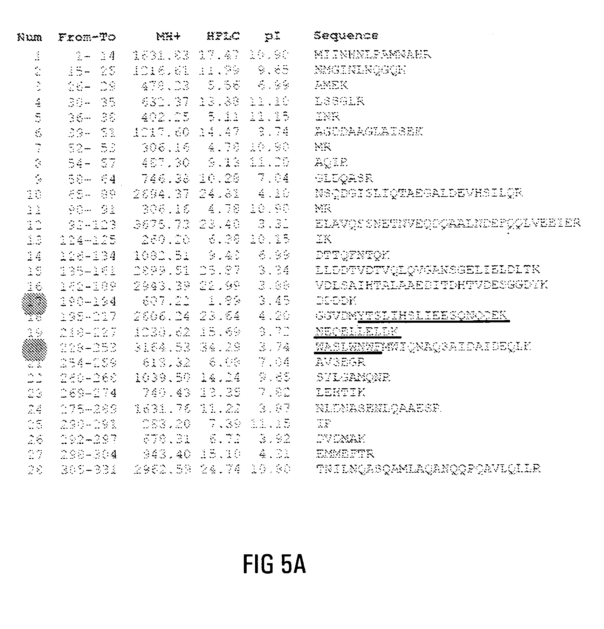

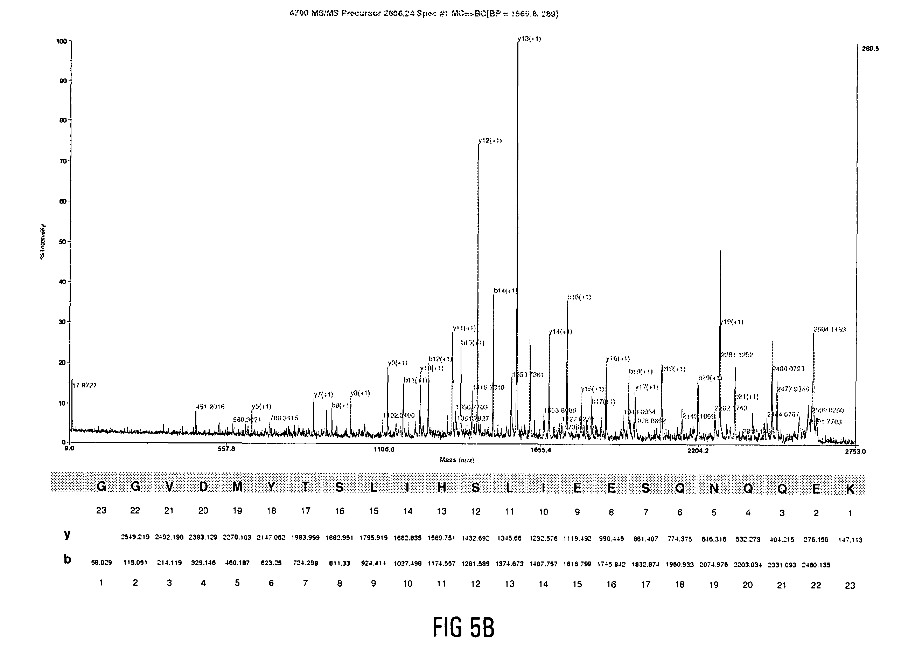

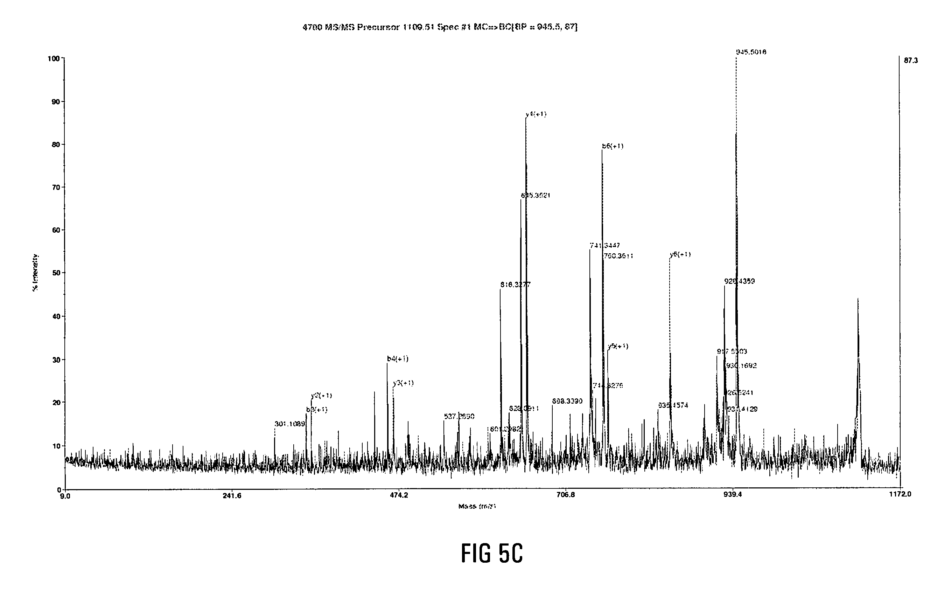

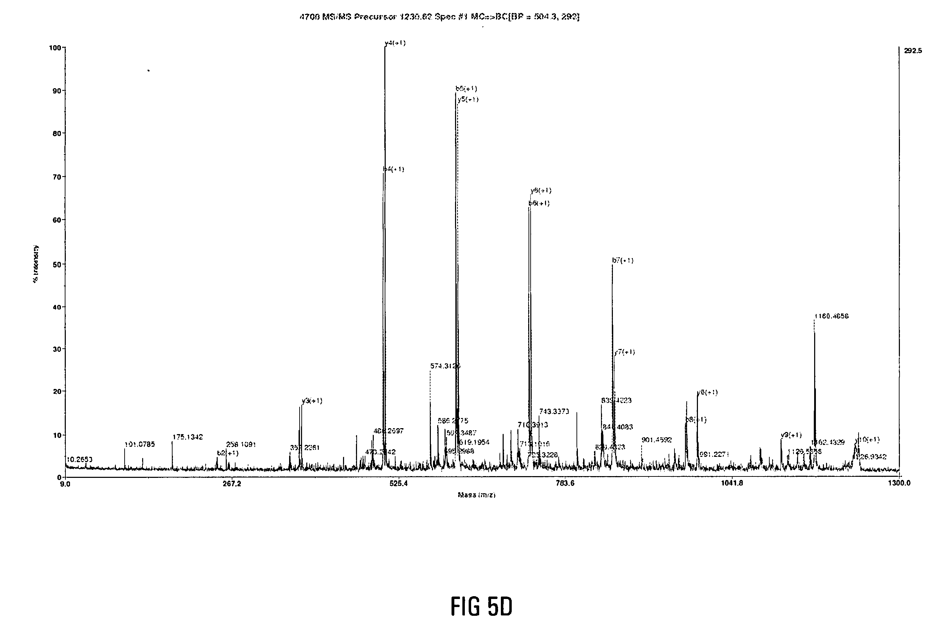

FIGURE 5: Mass spectrometry (MS)-based data for the verification of Fuzeon expression and integrity. (A) Cleavage report and identified peptides of the chimeric flagellin gene product of pSECNFFuzC7. The sequence of the peptide of interest, Fuzeon^{™}, is underlined. (B) MS/MS spectrum of the mass 2606.240 confirming the presence of GGVDMYTSLIHSLIEESQNQQEK, the N-terminal region of Fuzeon^{™} peptide. (C) MS/MS spectrum of the mass 1109.51 confirming the presence of WASLWNWF, the C-terminal end of Fuzeon peptide. (D) MS/MS spectrum of the mass at 1230.62 confirmed to be the fragment NEQELLELDK of the Fuzeon^{™} peptide;

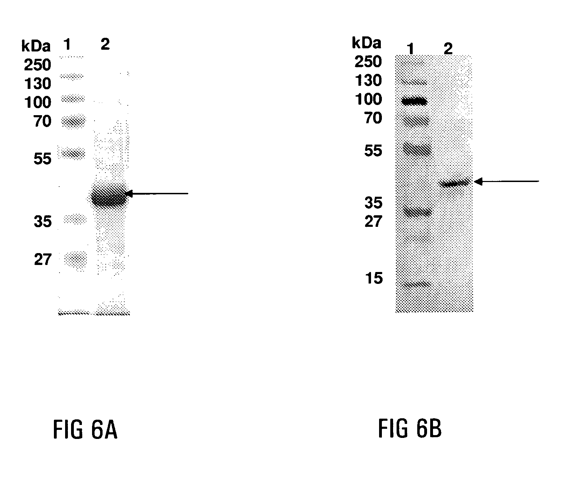

FIGURE 6A: SDS PAGE gel analysis of the extra-cellular protein fraction of chimeric flagellin carrying the FLAG-tag in *B*. *halodurans* strain BhFD05 after FLAG-affinity chromatography. Lane 1, low molecular mass ladder; lane 2, pSECNFINDC7. The arrow indicates the Indolicidin flagellin fusion protein;
FIGURE 6B: Western blot of the chimeric flagellin carrying the pSECNFINDC7 FLAG-tag. Lane 1, molecular weight marker (Fermentas); lane 2, pSECNFINDC7 FLAG-affinity eluate. The arrow indicates the Indolicidin flagellin fusion protein;

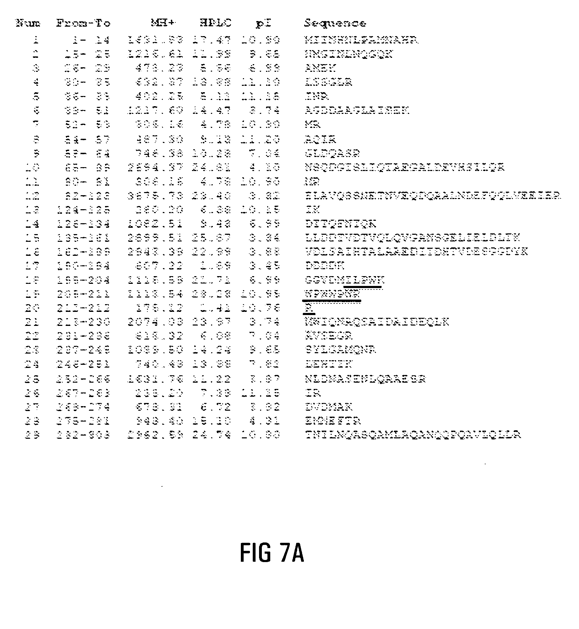

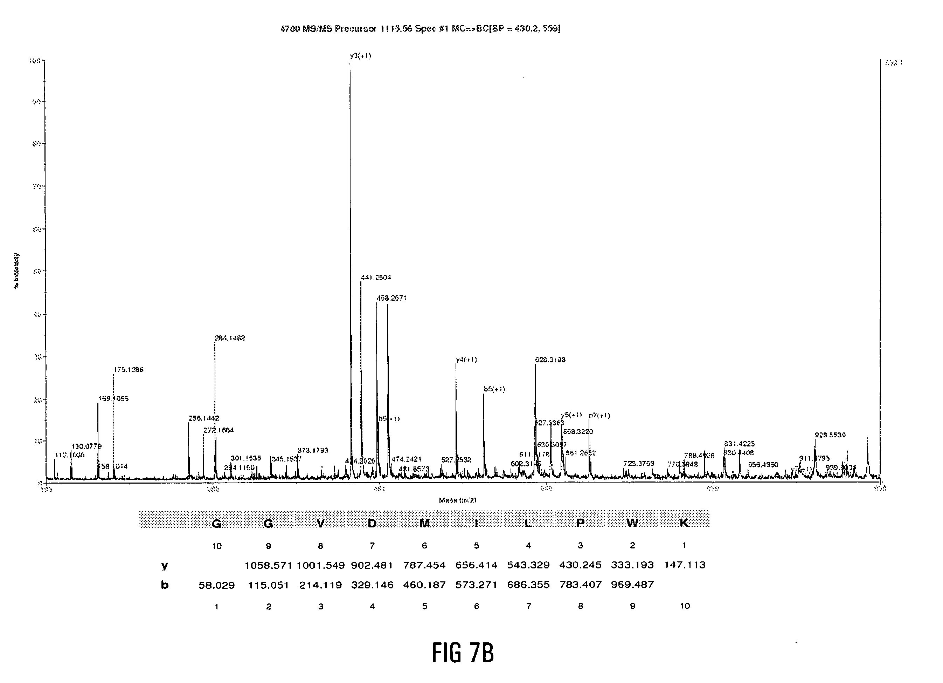

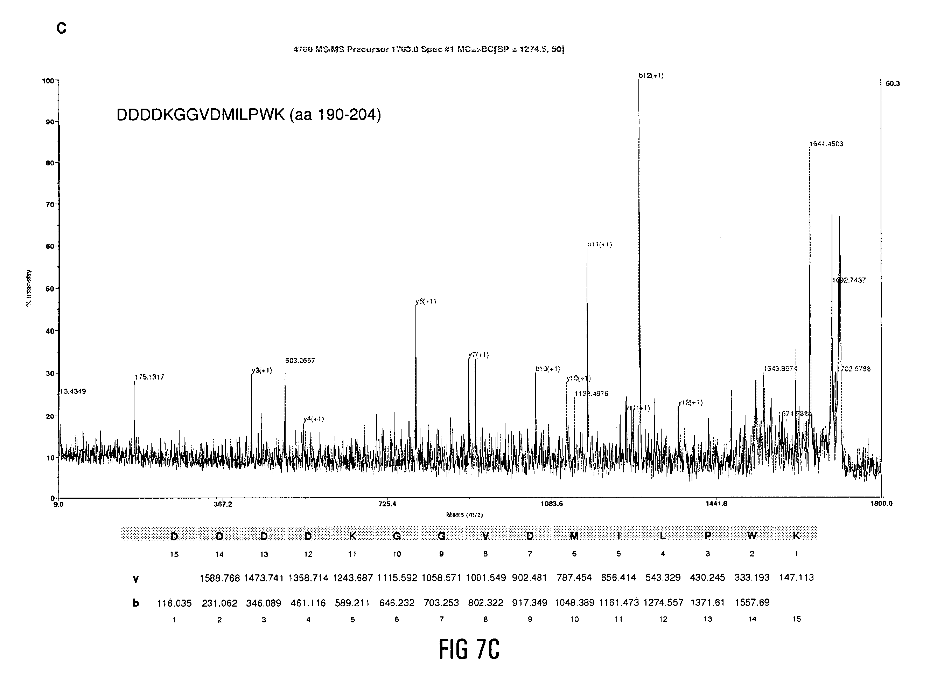

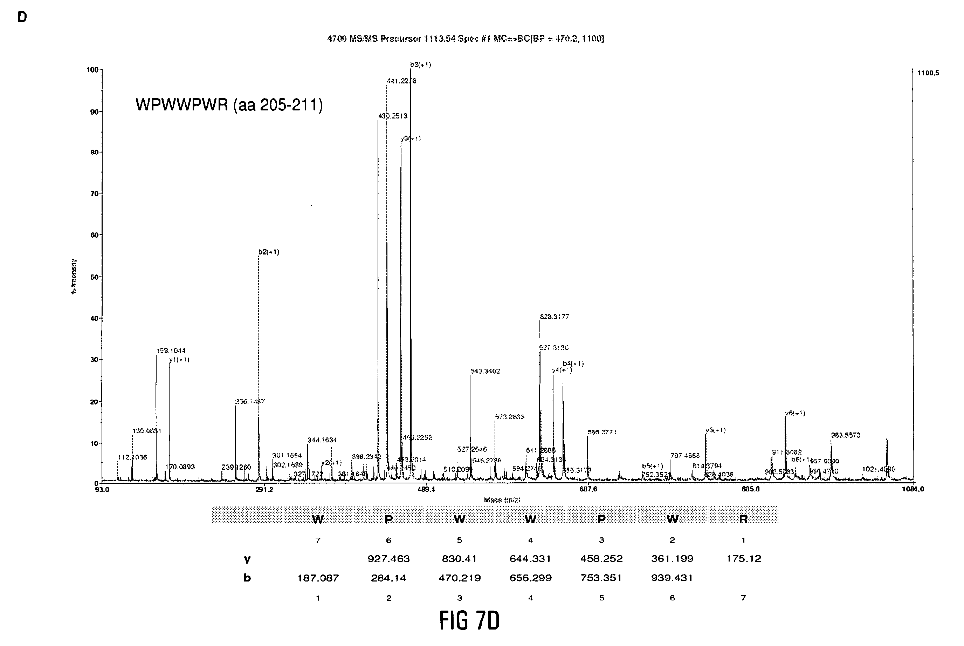

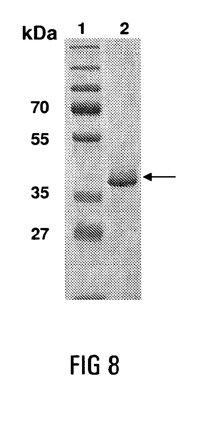

FIGURE 7: Mass spectrometry (MS)-based data for the verification of Indolicidin expression and integrity. (A) Cleavage report and identified peptides of the chimeric flagellin gene product of pSECNFINDC7. The sequence of the peptide of interest, Indolicidin, is underlined. (B) MS/MS spectrum of the mass 1115.59 Da confirming the presence of (GGVDMILPWK, aa 195-204), the N-terminal region of Indolicidin peptide. (C) MS/MS spectrum of the mass 1703.79 Da relating to the N-terminal part of Indolicidin, DDDDKGGVDMILPWK (aa 190-204) generated by a missed cleavage after K194, encompassing both of the tryptic peptides not clearly evident from the cleavage report shown in Fig. 8A. (D) MS/MS spectrum of the mass at 1113.542 confirming the presence of the peptide WPWWPWR (aa 205-211) of the Indolicidin peptide;
FIGURE 8: SDS-PAGE gel showing FLAG-tag purification of extra-cellular supernatant from strain BhFD05 (pSECNF2Sif) OD₆₀₀ 4.0. Lane 1, molecular weight marker (Fermentas); lane 2, FLAG-affinity eluate; The arrow indicates NF2Sif peptide;

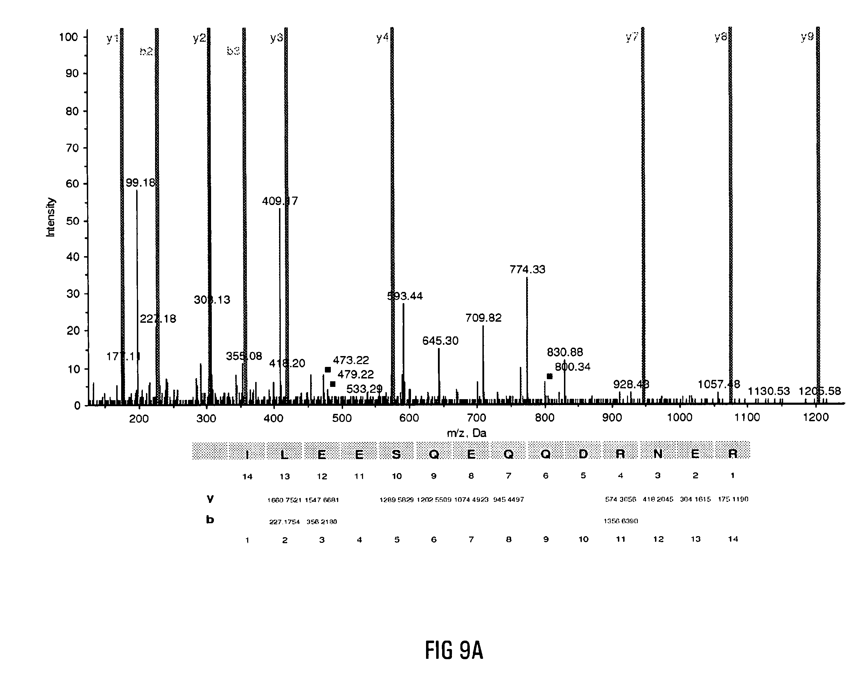

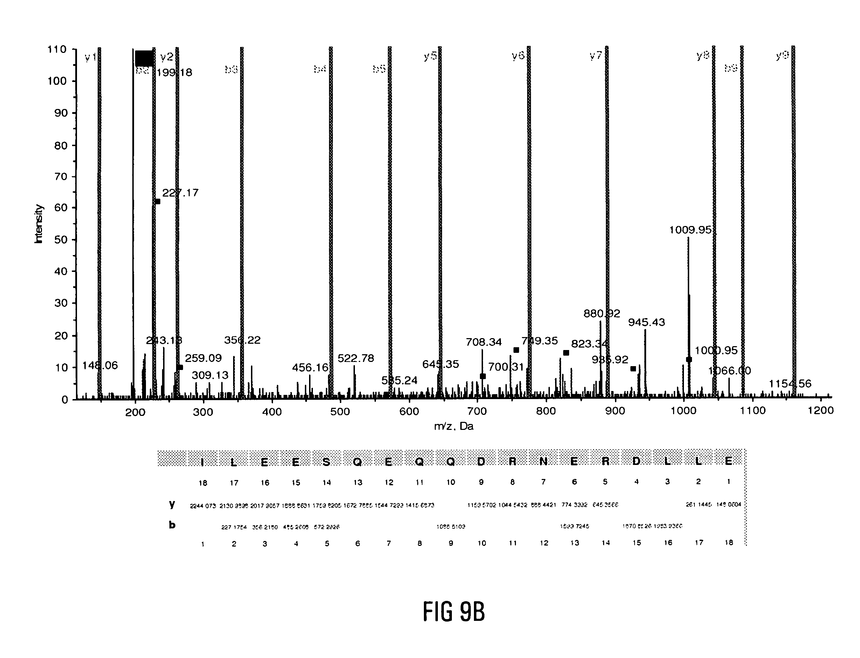

FIGURE 9: Mass spectrometry (MS)-based data for the verification of the chimeric flagellin gene product of pSECNF2Sif, Sifuvirtide. MS/MS spectra are indicated to confirm the presence of the masses at 1772.8372 Da, ILEESQEQQDRNER (A) and 2243.0657 Da, ILEESQEQQDRNERDLLE (B) for overlapping peptide sequences making up the C-terminal end of Sifuvirtide;

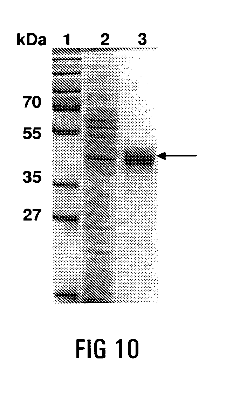

FIGURE 10: SDS-PAGE gel showing FLAG-tag purification of extra-cellular supernatant from strain BhFD05 (pSECNCF2Sif) OD₆₀₀ 4.0. Lane 1, molecular weight marker (Fermentas); lane 2, Crude sample and lane 3, FLAG-affinity eluate; The arrow indicates NCF2Sif peptide; and

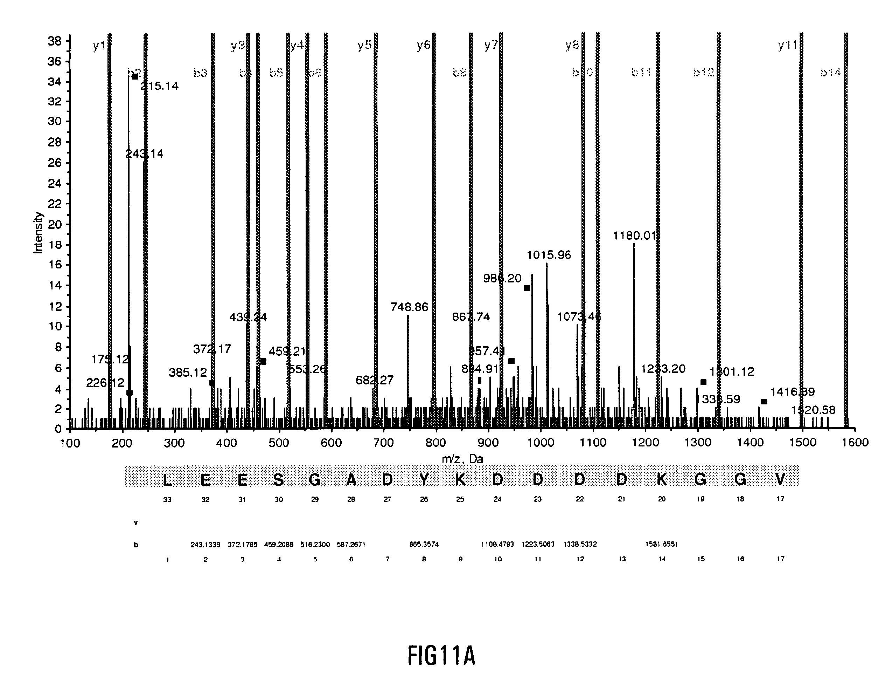

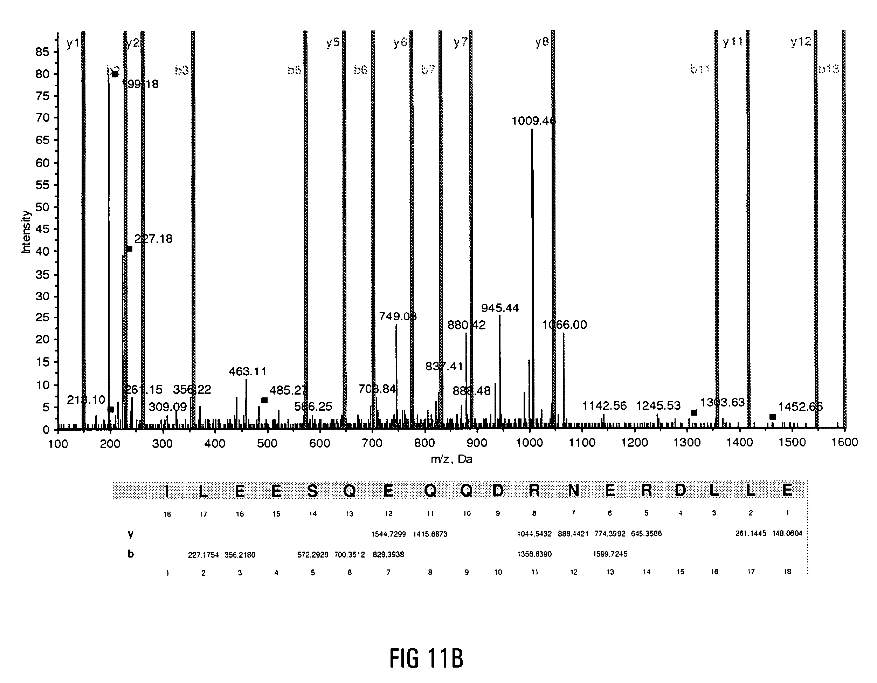

FIGURE 11: Mass spectrometry (MS)-based data for the verification of the chimeric flagellin gene product of pSECNCF2Sif, Sifuvirtide. MS/MS spectra are indicated to confirm the presence of the masses at 3938.6768 Da, the N-terminal region of the anti-viral peptide, LEESGADYKDDDDKGGVDMSWETWEREIENYTR (A); as well as 2243.0669 Da , the C-terminal region, ILEESQEQQDRNERDLLE (B).

### Development of host genetic background

### EXAMPLE 1:

### Inactivation of the fliD gene on the chromosome of B. halodurans BhFC04 (Δhag ΔwprA).

Primers were designed to amplify two fragments of the *fliD* gene by PCR amplification. These were 1.5 kb and 0.989 kb respectively and contained part of the N-terminal (primers δ*^{D}*Kpn / MC120805, Table1) and part of the C-terminal (primers FliDCF2/FliDCR2, Table1) regions of the *fliD* gene. The vector pSEC194 (Crampton et al. 2007) was digested with *Kpn*I/*Hind*II and ligated to both fragments in a 3 way ligation and transformed into *E*. *coli* DH10B to create the plasmid pSECFliD containing the defective *fliD* gene. This plasmid was then transformed into *B*. *halodurans* BhFC04 (Δ*hag*, Δ*wpr*A) and integration was according to Crampton et al (2007). Twenty putative single crossover colonies were screened with primers M13F and DChrRev (Table1). Five N-terminal single crossover clones were obtained and 15 C-terminal single crossover clones. One of the N-terminal crossover colonies was used to create a double crossover. PCR amplification with primers ChrFliFor and DChrRev (Table1) proved that the double crossover event did occur. Twenty chloramphenicol sensitive colonies were tested and 12 of them proved to be correct - containing the defective *fliD* gene while the rest were found to be revertants. This strain was named *B. halodurans* BhFD01 (Δ*hag, Δwpr*A, Δ*fliD*).

### EXAMPLE 2:

### Inactivation of key protease genes on the chromosome.

To further improve the stability of secreted chimeric peptide monomers by decreasing proteolytic degradation it was decided to inactivate proteases other than the *wpr*A cell wall protease on the *B. halodurans* BhFD01 chromosome. The protease genes targeted were: *alp* [SEQ ID NO. 3], *apr* [SEQ ID NO. 4], *asp* [SEQ ID NO. 6] and *vpr* [SEQ ID NO. 5].

### 2.1 Inactivation of the prepro-alkaline protease (alp) gene of B. halodurans Alk36 (BhFD01).

The *alp* gene is located at position 740001 to 741119 on the *B*. *halodurans* C125 genome (http://www.jamstec.co.jp/genomebase/micrhome). The primers used to generate the two PCR products *alp1* (alp1F/alp1R) and *alp2* (alp2F/alp2R) needed for construction of the defective *alp-* fragment are listed in Table 1. The 1183 bp *alp1* PCR product started 999 bps upstream of the ATG start codon and included the first 184 bps of the alp N-terminal region. The 848 bp *alp*2 PCR product included the last 161 bps of the *alp* C-terminal region as well as 687 bps downstream of the TAA stop codon of the *alp* gene.

**Table 1: List of PCR primers and their corresponding nucleotide sequences. Restriction enzyme sites are underlined.**

| **Primer name** | **Nucleotide sequence** | **Restriction enzyme sites** |
|---|---|---|
| alp1F [SEQ ID NO. 8] | 5' CTTGGTACCGCGTGGGAATGTTGCA 3' | *Kpn*I |
| alp1R [SEQ ID NO. 9] | 5' CTTGGATCCTGCACTTCTACCGCTGAG3' | *Bam*HI |
| alp2F [SEQ ID NO. 10] | 5' CTTGGATCCGGCTTCACCTCATGTGA 3' | *Bam*HI |
| alp2R [SEQ ID NO. 11] | 5' CTCCCGGGTGGTTGTCACAGCAGCGG 3' | *Sma*I |
| apr1F [SEQ ID NO. 12] | 5'GCAGGTACCGTTGGTGTTCAAGATGTTTACG-3' | *Kpn*I |
| apr1R [SEQ ID NO. 13] | 5'GCAGGATCCAGGCGTTGCTTGAGACGTACCA3' | *Bam*HI |
| apr2F [SEQ ID NO. 14] | 5'GCAGGATCCGGACAGGAAGCGAACCTCAAG3' | *Bam*HI |
| apr2R [SEQ ID NO. 15] | 5' GCACCCGGGCAAGTCCTAGAGTACAATAAC 3' | *Sma*I |
| vpr1F [SEQ ID NO. 16] | 5'CGTGTTACCGATGTGTAGTGCCTTATC3' | *Kpn*I |
| vpr1R [SEQ ID NO. 17] | 5'CTTGGATCCTTCATACGTCTCGCCATCGAG3' | *Bam*HI |
| vpr2F [SEQ ID NO. 18] | 5'CGTGGATCCCGAAGGTACGATCATCGTA3' | *Bam*HI |
| vpr2R [SEQ ID NO. 19] | 5'GTCCCGGGAAGCACGAGTGGATTCATGGTATA3' | *Sma*I |
| asp1F [SEQ ID NO. 20] | 5' GTAGGTACCCTCGATGCGAAAGTTCTCGATG 3' | *Kpn*I |
| asp1R [SEQ ID NO. 21] | 5' GCAGGATCCGTACCAGCCACGTGAGTTCCG 3' | *Bam*HI |
| asp2F [SEQ ID NO. 22] | 5' GCAGGATCCGCTAGATACTCTGGTGTTATGG 3' | *Bam*HI |
| asp2R [SEQ ID NO. 23] | 5' GATCCCGGGCCTCCTATCATACCCAAATGAG 3' | *Sma*I |
| MC120805 [SEQ ID NO. 24] | 5'CGAGGATCCCGTATTTAAAGAGGAACGTAA3' | *Bam*HI |
| FliDCF2 [SEQ ID NO. 25] | 5'CGAGGATCCCGAGCAGTGATTACAGATTG3' | *Bam*HI |
| FliDCR2[SEQ ID NO. 26] | 5'CGACCCGGGCAGAGAGCTCATTATGCTTCTC3' | *Sma*I |
| DChrRev [SEQ ID NO. 27] | 5'CTTAGATCATGGTTAGAATCAAGAGG3' | - |
| ChrFliFor [SEQ ID NO. 28] | 5'GCTTGTGCTGGGCAAAGGAGGCGAAG3' | - |
| δ^{D}Kpn [SEQ ID NO. 29] | 5'CTCGGTACCCTCGCGTTACGCTCTTTCTGT3' | *Kpn*I |
| FliNterRev [SEQ ID NO. 30] | 5'CTCCTCGAGCGACCTTCTGAAACAGC3' | *Xho*I |
| M13F [SEQ ID NO. 31] | 5'TGACCGGCAGCAAAATG3' | - |
| FliCR [SEQ ID NO. 32] | 5'CAACAAAGTAACGGTTGAGCG3' | - |
| FliDNR3 [SEQ ID NO. 33] | 5'CGAGGATCCAAGACCGGCAGAGTTAATGTC3' | *Bam*HI |
| NC5F [SEQ ID NO. 34] | | *Xho*I |
| VCF6 [SEQ ID NO. 35] | | *Sal*I |
| VNR6 [SEQ ID NO. 36] | 5'GACGTCGACAGTGTGGTCAGTAATATCCTC3' | *Sal*I |
| FliDCR [SEQ ID NO. 37] | 5'CGACCCGGGGAAGAAGCTGAAGACGATGCAGC3' | *Sma*I |
| FliC7F [SEQ ID NO. 38] | 5'CGAGGTACCAGGAGTTTGTCCTTCTG 3' | *Kpn*I |
| FuzendR [SEQ ID NO. 39] | 5'CGTCAGGATCCTTACATAAACCAATTCCA3' | *Bam*HI |
| FuzendR2 [SEQ ID NO. 40] | 5'GTCTAGGATCC | |
| TermF [SEQ ID NO. 41] | 5'GACGGATCCTTTGCTTCCATTTAAAGATCT3' | *Bam*HI |
| TermR [SEQ ID NO. 42] | 5'GTGCTGCAGGTATTTAAAGAGGAACGTAAACG3' | *Pst*I |
| SifRev [SEQ ID NO. 43] | 5'CTCGAGGATCCTTATTCTAATAAATCACGTTC3' | *Bam*HI |

The digested PCR products (N-terminal *Kpn*I/*Bam*HI and C-terminal *Bam*HI/*Sma*I) were ligated together into integration vector pSEC194 (*Kpn*I and *Hind*II) to obtain pSECalp- harbouring the defective *alp* gene with the internal 771 bps deleted.

The defective *alp*- gene was integrated into the *B. halodurans* BhFD01 (Δ *hag*, Δ*fliD* Δ *wpr*A) chromosome through a double crossover event as described in the previous section to create BhFD02 (Δ*hag*, Δ*fliD*, Δ*wpr*A, Δ*alp*). The event was confirmed through PCR analysis.

### 2.2 Inactivation of the prepro-alkaline protease (apr) gene of B. halodurans.

The prepro-alkaline protease (*apr*) gene is located at position 751087 to753465 on the *B*. *halodurans* C125 genome (http://www.jamstec.co.jp/genomebase/micrhome ). The primers used to obtain the two PCR products needed for construction of the defective *apr-*fragment are (apr1F/apr1R) and (apr2F/apr2R) (Table 1). The 1625 bp *apr1* PCR product started 644 bps upstream of the TTG start codon of the *apr* gene and included the first 981 bps of the N-terminal sequence. The 1235 bp *apr2* PCR product included the last 167 bps of the *apr* gene C-terminal sequence as well as 1068 bps downstream of the TAA stop codon of the *apr* gene

The *apr* PCR products (N-terminal *Kpn*I/*Bam*HI and C-terminal *Bam*HI/*Sma*I) were ligated together into the vector pSEC194 (*Kpn*I/*Hind*II) to obtain pSECapr- harbouring the defective *apr* gene with the internal 1231 bps deleted.

The defective *apr* gene was integrated into the *B. halodurans* BhFD02 (Δ*hag*, Δ*fliD*, Δ*wpr*A, Δ*alp*) chromosome through a double crossover event as described previously to create BhFD03 (Δ*hag*, Δ*fliD,* Δ*wpr*A, Δ*alp*, Δ*apr*). This event was confirmed through PCR analysis.

### 2.3 Inactivation of the vpr protease gene of B. halodurans.

The *vpr* gene is located at position 905382 to 902983 on the *B. halodurans* C125 genome (http://www.jamstec.co.jp/genomebase/micrhome). The primers used to obtain the two PCR products needed for construction of the *vpr-*fragment are summarized in Table 1 (vpr1F/vpr1R and vpr2F/vpr2R). The 1724 bp *vpr1* PCR product started 578 bps upstream of the TTG start codon of the *vpr* gene and included the first 1146 bps of the *vpr* N-terminal sequence. The 1509 bp *vpr2* PCR product included the last 361 bps of the *vpr* gene C-terminal sequence as well as 1148 bps downstream of the TAA stop codon of the *vpr* gene. The temperature sensitive pSEC194 was restricted with *Kpn*I and *Hinc*II and ligated to *vpr*1 and *vpr*2 to obtain pSECvpr.

The defective *vpr* gene was integrated into the *B. halodurans* BhFD03 (Δ*hag,* Δ*wpr*A, Δ*alp*, Δ*apr*) chromosome through a double crossover event as described previously. This event was confirmed through PCR analysis and created BhFD04 (Δ*hag*, Δ*fliD*, Δ*wpr*A, Δ*alp*, Δ*apr*, Δ*vpr*).

### 2.4 Inactivation of the asp protease gene of B. halodurans.

The asp gene is located at position 927497 to 928582 on the *B. halodurans* C125 genome (http://www.jamstec.co.jp/genomebase/micrhome ). The primers used to generate the two PCR products *asp1* (asp1F/asp1R) and asp2 (asp2F/asp2R) needed for construction of the *asp*- fragment are listed in Table 1. The 852 bp *asp1* PCR product started 371 bps upstream of the ATG start codon and included the first 481 bps of the *asp* N-terminal region. The 682 bp *asp2* PCR product included the last 319 bps of the *asp* C-terminal region as well as 363 bps downstream of the TAA stop codon of the *asp* gene. The digested PCR products (N-terminal *Kpn*I/*Bam*HI and C-terminal *Bam*HI/*Sma*I) were ligated together into integration vector pSEC194 (*Kpn*I and *Hind*II) to obtain pSECasp - harbouring the defective *asp* gene with the internal 281 bps deleted. The defective asp gene was integrated into the *B*. *halodurans* BhFD04 (Δ*hag*, Δ*fliD*, Δ*wprA*, Δ*alp*, Δ*apr*, Δ*vpr*) chromosome through a double crossover event as described in the previous sections to obtain strain BhFD05 (Δ*hag*, Δ*fliD*, Δ*wpr*A, Δ*alp*, Δ*apr*, Δ*vpr*, Δ*asp*). This event was confirmed through PCR analysis.

### Application of host strain: Peptides secreted according to the invention as in-frame flagellin sandwich fusions.

### EXAMPLE 3:

The effect of the *fliD* mutation on the secretion of chimeric flagellin fusions was evaluated with the following different peptides.
i) An HIV antigenic peptide (Table 2) of which the synthetic peptide sequence used was based on a consensus sequence of the variable region of all HIV-1 subtype C V3 South African isolates, Hewer and Meyer (2003). This peptide is 24 amino acids in size and the full insert size is 29 amino acids. The amino acid sequence of FliC including the HIV-antigenic peptide is provided as SEQ ID NO: 44. The construct which encodes the polypeptide sequence of SEQ ID NO: 44 was named pSECNHIVC7 and is provided as SEQ ID NO: 45.
ii) Indolicidin a cationic antimicrobial peptide (Table 2) of 13 amino acids (Hancock and Lehrer, 1998) was inserted as an in-frame chimeric flagellin fusion with a FLAG-tag inserted to facilitate isolation. The full insert size was 36 amino acids, which included methionine residues inserted either side of the Indolicidin peptide for cyanylation and selective cleavage commonly performed with cyanogen bromide (CnBr) (Tang and Speicher 2004). The amino acid sequence of FliC including the Indolicidin insert, with a FLAG-tag and cleavage sites, is provided as SEQ ID NO: 46. The construct which encodes the polypeptide sequence of SEQ ID NO: 46 was named pSECNFINDC7, and is provided as SEQ ID NO: 47.
iii) Enfuviritide is an HIV-fusion inhibitor that is marketed as Fuzeon^{™} and the synthetic oligonucleotides chosen were based on the amino acid sequence of enfuvirtide (Table 2) (Bolognesi et al. 1995). The peptide is 36 amino acids in size and a FLAG-tag was included to facilitate isolation as were methionine residues which were inserted either side of the peptide to facilitate cleavage. The total number of amino acids inserted into this construct was 59. The amino acid sequence of FliC including the enfuviritide insert, with a FLAG-tag and cleavage sites, is provided as SEQ ID NO: 48. The construct which encodes the polypeptide sequence of SEQ ID NO: 48 was named pSECNFFuzC7 and is provided as SEQ ID NO: 49.
iv) Sifuvirtide a 36 amino acid peptide (Table 2) is a promising alternative to enfuvirtide as it shows improved activity and stability (Franquelim et al. 2008). Methionine cleavage sites as well as two FLAG-tags were included giving a full insert size of 75 amino acids. The amino acid sequence of FliC including the Sifuviritide insert, with two FLAG-tags and cleavage sites, is provided as SEQ ID NO: 50. The construct which encodes the polypeptide sequence of SEQ ID NO: 50 was named pSECNF2SifC7 and is provided as SEQ ID NO: 51.

**Table 2. HIV clade C, FLAG-Indolicidin, FLAG-Fuzeon and FLAGx2-Sifuvirtide polypeptides inserted into the NC7 site of the flagellin protein, the FLAGx2-Sifuvirtide polypeptide ligated to the N-terminal region of the flagellin protein and the FLAG-Sifuvirtide polypeptide ligated to the C-terminal of the flagellin protein**

| **Peptide Name** | **Peptide sequences** |
|---|---|
| HIV antigenic peptide [SEQ ID NO. 52] | **TRPNNNTRKSIRIGPGQTFYATGD** |
| FLAG-Indolicidin (NFINDC7) [SEQ ID NO. 53] | **DYKDDDDK**GGVDM**ILPWKWPWWPWRR**M |
| FLAG-Fuzeon (NFFuzC7) [SEQ ID NO. 54] | |
| FLAGx2-Sifuvirtide (NF2SifC7) [SEQ ID NO. 55] | |
| FLAGx2-Sifuvirtide (NF2Sif) ligated to N-terminal [SEQ ID NO. 56] | |
| FLAG-Sifuvirtide (NCFSif) ligated to C-terminal [SEQ ID NO. 57] | |

Bold amino acids indicate polypeptide and FLAG sequences, non-bold amino acids code for linkers and the shaded amino acids indicate the inserted methionine residues.

**Table. 3. List of complimentary oligonucleotide sequences used in the construction of Indolicidin, Fuzeon^{™}, and Sifuvirtide expression cassettes.**

| **Primer Name** | **Oligonucleotide sequences** | **Restriction sites** |
|---|---|---|
| HIVF [SEQ ID NO. 58] | | *Sal*I |
| HIVR [SEQ ID NO. 59] | | *Bam*HI |
| IndF [SEQ ID NO. 60] | | *Sal*I |
| IndR [SEQ ID NO. 61] | | *Sal*I |
| FuzF [SEQ ID NO. 62] | | *Sal*I |
| FuzR [SEQ ID NO. 63] | | *Bam*HI |
| SifF [SEQ ID NO. 64] | | *Saf*I |
| SifR [SEQ ID NO. 65] | | *Bam*HI |
| FLAGF [SEQ ID NO. 66] | | *Sal*I |
| FLAGR [SEQ ID NO. 67] | | *Sal*I |

Bold nucleotides indicate polypeptides and FLAG sequences, non bold nucleotides code for the polylinker sequences and the shaded nucleotides indicate the inserted methionine residues. The underlined nucleotides correspond to the restriction enzyme sites.

These peptides were all inserted in the central variable region of the flagellin peptide monomer within the polylinker which was inserted at nucleoside position 540 in the flagellin gene (Crampton et al. 2007).

### 3.1 Construction of pSECNC7 cassette.

This construct was an enhancement of construct pSECNC6 (described in PCT International Application PCT/IB2005/054022; International publication no. WO 2006/072845). The cassette pSECNC7 was obtained by PCR amplification of the N- (1447 bp) and C-terminal (1401 bp) regions of the flagellin gene with primers FliC7F/VNR6 and FliDNR3/VCF6 respectively (Table 1). The N-terminal and C-terminal products were digested with *KpnI*/*SalI* and *Sal*I respectively. Both fragments were ligated into pSEC194 (*Kpn*I/*Hinc*II) in a three way ligation to obtain pSECNC7 which contains the 27 base pair polylinker at nucleotide position 540 (Fig. 1).

### 3.2 Evaluation of the expression of immunogenic peptides.

### 3.2.1 Construction and evaluation of immunogenic chimeric flagellin fusion pSECNHIVC7 secreted from B. halodurans BhFD05.

The plasmid pSECNHIVC7 (Table 2 and Table 3) containing the antigenic HIV peptide, construct as described by Crampton et al. (2007) was used and transformed into all five protease deficient strains; *B. halodurans* BhFD01, BhFD02, BhFD03, BhFD04 and BhFD05 and evaluated for secretion of the fusion peptide into the extracellular medium.

The chimeric flagellin gene product of pSECNHIVC7 in the different host backgrounds were evaluated for secretion into the extracellular medium. Cells were grown to an OD₆₀₀ of between 3.5 and 5.5 before harvesting. Approximately 1.5 ml of cell culture was harvested at 8000 X g for 1 minute (supernatant contained extracellular fraction). 1 ml of the supernatant was removed and placed in an Eppendorf tube. 330 µl of a 20% TCA (trichloroacteic acid) solution was added to the supernatant and incubated on ice with shaking for 30 minutes. Extracellular proteins were pelleted at 12000 X g for 10 minutes. The pellet was washed once with an ethanol and ether solution (1::1 v/v). Pellets were dried and proteins were resuspended in 30 µl 25 mM Tris-HCl solution (pH 9.5).

The extracellular protein fractions (10 µl) were analysed on a 10% SDS-PAGE gels and visualized using colloidal Coomassie stain (Fig. 2A). Approximately 1-2 µg was used for Western blot analysis (Fig. 2B)

### 3.2.2 Analysis of immunogenic chimeric flagellin fusions secreted from B. halodurans BhFD05.

Cells were grown to an OD₆₀₀ of between 3.5 and 5.5 at 1 litre scale before harvesting the supernatant by centrifugation at 8000 X g for 10 minutes. Total extracellular protein was precipitated from the supernatant by the addition of 10% (w/v) TCA (trichloroacteic acid) and incubation on ice for 30 minutes. Extracellular proteins were pelleted at 12000 X g for 10 minutes. The pellet was washed once with an ethanol and ether solution (1:1 v/v). Pellets were air dried and proteins were resuspended in 10 ml 50 mM Tris-HCl solution (pH 7.4). The immunogenic chimeric flagellin fusion protein was purified to near homogeneity from the crude total extracellular protein fraction by anion exchange chromatography. The entire 10 ml crude protein preparation was loaded onto Toyopearl 650M strong anion exchange resin (Tosoh Bioscience) in a 1.6 x 13 cm pre-packed column using an ÄKTA FPLC^{™} protein purification system. Contaminating non-protein components were washed off the resin by 10-15 column volumes of wash buffer (50 mM Tris-HCl pH 7.4) until the baseline absorbance (A₂₈₀ₙₘ) stabilized around zero. Proteins were eluted off the resin by an increasing NaCl gradient ranging from 0-500 mM NaCl in 50 mM Tris-HCl, pH 7.4 over 15 column volumes. Protein-containing elution fractions were analysed on 10% SDS-PAGE gels to assess the final purity of the immunogenic chimeric flagellin fusion protein.

The purified immunogenic chimeric flagellin fusion protein was quantified using a Qubit^{™} fluorometer (Invitrogen) with Quant-iT^{™} protein assay reagents containing a highly sensitive protein-specific fluorescent dye and certified protein standards, used according to the manufacturer's instructions. The total protein quantity obtained was 9.87 mg immunogenic chimeric flagellin fusion protein per litre culture supernatant, which relates to ~9.38 mg/L at an estimated 95% purity. After purification the chimeric flagellin fusion protein was analyzed on 10% SDS-PAGE gels as described above (Fig. 3A). Approximately 1-2 µg was used for Western blot analysis using using MEIV3b 4 antibodies (Fig. 3B).

### 3.3 Evaluation of the expression of anti-viral peptides Fuzeon^{™} and Sifuvirtide.

### 3.3.1 Construction of pSECNFFuzC7 carrying the FLAG::Fuzeon peptide.

The synthetic oligonucleotides (Table 3) were designed based on the enfuvirtide (Fuzeon^{™}) amino acid sequence (Table 2) (Bolognesi et al. 1995). A methionine residue was included at the N- and C-terminal end for chemical cleavage using cyanogen bromide (Tang and Speicher. 2004). A FLAG-tag was incorporated for ease of purification.

Oligonucleotides (Table 1) FuzF and FuzR were annealed according to the method described by IDT (Integrated DNA Technologies, wwv.idtdna.com). The oligonucleotides were diluted in STE buffer (10 mM Tris pH 8, 50 mM NaCl, 1 mM EDTA) to a final concentration of 20 µM. A working stock (5 µM) was made and equal amounts of complimentary oligonucleotides were mixed together (usually 25 µl of each). Samples were boiled for 5 minutes and allowed to cool very slowly in the waterbath. The resulting product was restricted with the appropriate restriction enzymes and ligated into pSECNC7 digested with *Sal*I and BamHI. The resulting construct was named pSECNFuzC7. This construct was digested with *Sal*I. FLAG-tag oligonucleotides were derived from the peptide sequence DYKDDDDK (Table 2) (Sigma cat no F3290)) with addition of a 4 and 2 amino acid linker at the N- and C-terminal ends for better FLAG-tag exposure. FLAG-tag oligonucleotides (Table 3) were annealed as described above. The annealed oligonucleotides were ligated to pSECNFuzC7 digested with *Sal*I to obtain pSECNFFuzC7.

All constructs were confirmed to be correct by PCR analysis using primers FliNterRev and NC5F (Table 1). Only after confirmation of construct integrity were they transformed into *B. halodurans* BhFD05 using the modified protoplast transformation method (Crampton et al. 2007). Orientation of the insert was confirmed with restriction digests and directional PCR using primers FliCR and FuzF (Table 1 and Table 3).

### 3.3.2 Construction of pSECNF2SifC7 carrying a FLAGx2::Sifuvirtide peptide.

The synthetic oligonucleotides were derived from the Sifuvirtide peptide sequence (Table 2) (Franquelim et al. 2008) and included a methionine residue at the N- and C-terminal ends for cyanogen bromide chemical cleavage. Oligonucleotides SifF and SifR (Table 3) were annealed according to the method described in section 3.3.1. The resulting product was restricted with the appropriate restriction enzymes (Table 1) and ligated into pSECNC7 digested with *Sal*I and *Bam*HI. The resulting construct was named pSECNSifC7.

pSECNSifC7 was digested with *Sal*I. FLAG-tag oligonucleotides were annealed as described in section 3.3.1. The annealed oligonucleotides were ligated to pSECNSifC7 and resulted in the incorporation of two FLAG-tags in front of the Sif peptide. This construct was named pSECNF2SifC7.

All constructs were confirmed to be correct by PCR and sequencing analysis as described for pSECNFFuzC7. Orientation of the insert was confirmed by directional PCR using primers FliCR and SifF (Table 1 and Table 3). Constructs were then transformed into *B. halodurans* BhFD05.

### 3.3.3 Evaluation of anti-viral chimeric flagellin fusions secreted from B. halodurans BhFD05.

The chimeric flagellin gene products of pSECNFFuzC7 and pSECNF2SifC7 were evaluated for secretion into the extracellular medium. Cells were grown to an OD₆₀₀ of between 3.5 and 5.5 before harvesting. Approximately 1.5 ml of cell culture was harvested at 8000 X g for 1 minute (supernatant contained extracellular fraction). 1 ml of the supernatant was removed and placed in an Eppendorf tube. 330 µl of a 20% TCA (trichloroacteic acid) solution was added to the supernatant and incubated on ice for 30 minutes. Extracellular proteins were pelleted at 12000 X g for 10 minutes. The pellet was washed once with an ethanol and ether solution (1:1 v/v). Pellets were dried and proteins were resuspended in 30 µl 25 mM Tris-HCl solution (pH 9.5).

The extracellular protein fractions (10 µl) were analysed on a 10% SDS-PAGE gels and visualized using colloidal Coomassie stain. There were detectable chimeric flagellin protein bands for the flagellin fusions at the size range of 45 kDa (results not shown).

### 3.3.4 Analysis of anti-viral chimeric flagellin fusions secreted from B. halodurans BhFD05.

The desired flagellin-fusion protein was purified using affinity chromatography. The term "affinity chromatography" refers to a technique for separating molecules by their affinity to bind ligands attached to an insoluble matrix, so that the bound molecules can subsequently be eluted in a relatively pure state. The technique involved attaching a FLAG-tag to the fusion peptide, performing the chromatographic separation and isolating the fusion protein by excision from a (10%) SDS-PAGE gel.

The chimeric flagellin gene products of pSECNFFuzC7 and pSECNF2SifC7 were evaluated for secretion into the extracellular medium. Cells were grown to an OD₆₀₀ of between 3.5 and 5.5 at 1 litre scale before harvesting the supernatant by centrifugation at 8000 X g for 10 minutes. Total extracellular protein was precipitated from the supernatant by the addition of 10% (w/v) TCA (trichloroacteic acid) and incubation on ice for 30 minutes. Extracellular proteins were pelleted at 12000 X g for 10 minutes. The pellet was washed once with an ethanol and ether solution (1:1 v/v). Pellets were air dried and proteins were resuspended in 10 ml 50 mM Tris-HCl solution (pH 7.4) containing 500 mM NaCl and 2.5% (v/v) Triton X-100. The crude protein solution was incubated with 1 ml M2 Anti-FLAG affinity resin (Sigma) at room temperature for 2 hours to bind the FLAG-tagged fusion protein of interest. Non-specifically bound proteins were removed by washing the resin with 15 ml 50 mM Tris-HCl solution (pH 7.4) containing 500 mM NaCl and 2.5% (v/v) Triton X-100. The FLAG-tagged fusion protein was eluted with 8 ml 0.1M Glycine, pH 3.5 and analyzed on 10% SDS-PAGE gels as described in section 3.2.3 above (Fig. 4A). Approximately 1-2 µg was used for Western blot analysis using polyclonal rabbit anti-flagellin antibodies (Fig. 4B). The purified immunogenic chimeric flagellin fusion protein was quantified using a Qubit^{™} fluorometer (Invitrogen) with Quant-iT^{™} protein assay reagents containing a highly sensitive protein-specific fluorescent dye and certified protein standards, used according to the manufacturer's instructions. The total protein quantity obtained ranged between 10 and 20 mg immunogenic chimeric flagellin fusion protein per litre of culture supernatant, at an estimated 95% purity.

The anti-viral chimeric flagellin::Fuzeon protein band obtained from the pSECNFFuzC7 sample was excised from SDS-PAGE gels and subjected to enzymatic cleavage by modified sequencing grade trypsin through a method well known by those of skill in the art (Shevchenco and Shevchenco. 2001). This cleavage procedure cleaves polypeptides at the C-terminal side of lysine (K) and arginine (R) residues. The Fuzeon^{™} peptide presence and integrity were verified by mass-spectrometry analysis on an Applied Biosystems/MDS SCIEX 4800 MALDI TOF/TOF analyzer with CHCA (alpha-cyano-4-hydroxycinnamic acid) as the matrix and 1 fmol bradykinin as an internal calibrant (protonated, monoisotopic mass of 1060.5692). A preliminary examination of the mass spectra taken in reflector positive mode over the mass range 600-4000 Da, indicates that many of the highest peaks in each spectrum correspond to the tryptic peptides expected for the construct. A review of the cleavage report confirms that all expected masses within the range used in this mode (600-4000 Da) are present except for peptides 17 and 20 (Fig. 5A). Peptide mass matching was performed using GPMAW 7.1 with a precision of 50 ppm to check the coverage of the sequence in more detail. Twenty five masses were matched to 26 unmodified peptides, and 8 masses to 8 modified peptides, with 85% of the residues covered (284 of 331 amino acids). Each of these three precursors provided fairly complete y- and b-ion series. In order to further verify the sequence, several MS/MS spectra were acquired. Annotated MS/MS spectra are indicated for the peptides of interest with mass labels, y-ions and b-ions identified (Fig. 5B, C, D). The peptide of interest spans the tryptic peptides expected at 2606.24, 1230.62, and 3164.53 Da (Fig. 5A). The peak at 2606.240 was identified as the peptide GGVDMYTSLIHSLIEESQNQQEK (aa 195-217, Fig. 5B) corresponding to the N-terminal region of Fuzeon^{™}. No peak was evident at 3164.53 Da, but there was a peak at 1109.52 corresponding to the C-terminal portion of the inserted sequence. One peak in the MS spectrum stood out as a mass at 2074.03. An MS/MS spectrum of this mass was easily assigned to the peptide MWIQNAQSAIDAIDEQLK. This peptide does indeed occur in the fusion sequence, just after the inserted peptide of interest, and accounts for most of the peptide not covered by mass matching. The other half of the expected peptide should occur at 3164.53Da - 2074.03Da +18Da +1Da = 1109.5Da, where a peak of significant intensity was indeed present. This peak was confirmed by MS/MS to correspond to WASLWNWF (Fig. 5C), the C-terminal end of the Fuzeon^{™} peptide of interest. Furthermore, the mass at 1230.62 was confirmed to be NEQELLELDK (Fig. 5D) of the peptide of interest. In summary, there is positive proof that the peptide of interest, Fuzeon, (underlined, Fig. 5A) is indeed being expressed in its entirety as a chimeric flagellin fusion.

### 3.4 Evaluation of the expression of antimicrobial peptide Indolicidin.

### 3.4.1 Construction of pSECNFINDC7 carrying the FLAG::Indolicidin peptide.

Synthetic Indolicidin oligonucleotides were derived from Selsted et al. (1992) and included a methionine residue at the N- and C- terminal ends for chemical cleavage (Table 2). Oligonucleotides (Table 3) were annealed as described in section 3.3.1. The resulting product was restricted with the appropriate restriction enzymes and ligated into pSECNC7 digested with *Sal*I and *Bam*HI resulting in pSECNINDC7. This construct was digested with *Sal*I. FLAG-tag oligonucleotides were annealed as described in section 3.3.1. The annealed oligonucleotides were ligated to pSECNINDC7 and resulted in the incorporation of one FLAG-tag in front of the IND peptide. This construct was named pSECNFINDC7. The constructs were confirmed to be correct by PCR and sequencing analysis and transformed into *B. halodurans* BhFD05.

### 3.4.2 Analysis of antimicrobial chimeric flagellin fusions secreted from B. halodurans BhFD05.

The antimicrobial peptide, Indolicidin, was extracted and purified from *B. halodurans* culture supernatant as described in section 3.3.4. The FLAG-tagged fusion protein of interest was isolated by affinity chromatography. The purified immunogenic chimeric flagellin fusion protein was quantified using a Qubit^{™} fluorometer (Invitrogen) with Quant-iT^{™} protein assay reagents containing a highly sensitive protein-specific fluorescent dye and certified protein standards, used according to the manufacturer's instructions. The total protein quantity obtained was between 1 and 5 mg immunogenic chimeric flagellin fusion protein per litre of culture supernatant, at an estimated 95% purity. Samples were run on a SDS-PAGE (10%) gel (Fig. 6A) and Western blot analysis using polyclonal rabbit anti-flagellin antibodies confirmed the results (Fig. 6B). The anti-microbial chimeric flagellin::Indolicidin protein band was excised from SDS-PAGE (10%) gels and subjected to cleavage by modified sequencing grade trypsin and MALDI TOF/TOF mass spectrometry analysis as described in section 3.3.4 above. A review of the cleavage report confirms that all expected masses within the range used in reflector positive MS mode (600-4000 Da) are present, except for peptides 17 and 18 (Fig. 7A). Peptide mass matching was performed using GPMAW 7.1 with a precision of 50 ppm to check the sequence coverage in more detail. 42 masses were matched to 39 unmodified peptides, and 17 masses to 16 modified peptides, with 99% of the residues covered (306 of 308 amino acids). In order to verify that the peptides of interest are being expressed in the fusion constructs, MS/MS spectra were obtained for several masses in the regions of interest. Annotated MS/MS spectra are indicated for the peptides of interest with mass labels, y-ions and b-ions identified (Fig. 7B, C, D). The peptide expected at 1115.59 Da (GGVDMILPWK, amino acids 195-204) was identified and includes the N-terminal portion of the peptide of interest (Fig. 7B). To further demonstrate the presence of the N-terminal portion of the inserted sequence, an MS/MS spectrum was acquired of the precursor mass at 1703.79 Da (Fig. 7C). The identified peaks provide evidence for the peptide DDDDKGGVDMILPWK (amino acids 190-204) generated by a missed cleavage after K194, and encompassed both of the tryptic peptides not clearly evident from the cleavage report shown in Fig. 7A. The MS/MS spectrum of the precursor mass of 1113.542 confirming the presence of the peptide WPWWPWR (amino acids 205-211) is indicated in Fig. 7D. This peptide makes up the C-terminal region of Indolicidin. In summary, there is positive proof that the peptide of interest, Indolicidin, is indeed expressed in it's entirety as a chimeric flagellin fusion.

### Application of host strain: Peptides secreted according to the invention as N-terminal flagellin fusions.

### EXAMPLE 4:

### 4.1 Expression as FliC N-terminal fusion of the anti-viral peptide, Sifuvirtide.

### 4.1.1 Construction of pSECNF2Sif carrying the FLAG::Sifuvirtide peptide.

The plasmid pSECNF2SifC7 was used as template for the primers *σ^{D}*Kpn and SifRev2 to obtain a 947 bp PCR product containing the FliC N-terminal fragment (770 bp) fused to the 2xFLAG::Sifuvirtide peptide (225 bp) without a methionine residue at the C-terminal side of the Sifuvirtide peptide and the inclusion of a stop codon. The encoded polypeptide sequence of the FliC N-terminal fragment including the Sifuvirtide insert as described is provided as SEQ ID NO: 68. The same plasmid was used as a template with primers TermF and TermR to obtain a PCR product containing the FliC 3' untranslated region (700 bp). These two fragments were digested with appropriate enzymes and ligated to pSEC194 digested with *Kpn*I and *Hin*dII. The resulting construct, which encodes for the polypeptide sequence of SEQ ID NO: 68, was named pSECNF2Sif and is provided as SEQ ID NO: 69. The constructs were confirmed to be correct by PCR and sequencing analysis as described for pSECNFFuzC7 and then transformed into *B. halodurans* BhFD05.

### 4.1.2 Evaluation of the expression of the N-terminal peptide fusion.

The chimeric flagellin gene product of pSECNF2Sif was evaluated for secretion into the extracellular medium of *B. halodurans* BhFD05 according to section 3.3.3.

The extracellular protein fraction was analysed on a 10% SDS-PAGE gel. There was a detectable chimeric flagellin protein band for the flagellin::Sifuvirtide fusion at the size range of ~38 kDa. The antiviral peptide FLAGx2-Sifuvirtide, was extracted and purified from *B. halodurans* BhFD05 culture supernatant as described in section 3.3.4. Samples were run on a 10% SDS-PAGE gel (Fig. 8).

### 4.1.3 Analysis of the N-terminal peptide fusion.

The purified immunogenic chimeric flagellin fusion protein was quantified using a Qubit^{™} fluorometer (Invitrogen) with Quant-iT^{™} protein assay reagents containing a highly sensitive protein-specific fluorescent dye and certified protein standards, used according to the manufacturer's instructions. The total protein quantity obtained ranged between 1 and 5 mg immunogenic chimeric flagellin fusion protein per litre of culture supernatant, at an estimated 95% purity. The anti-viral chimeric flagellin fusion protein was electrophoresed on a 10% SDS-PAGE gel and bands were excised from the gel and subjected to enzymatic cleavage by modified sequencing grade trypsin through a method well known by those of skill in the art. The Sifuvirtide peptide presence and integrity were verified by mass-spectrometry analysis using a QSTAR^{®} Elite mass spectrometer (Applied Biosystems/MDS SCIEX) with a discrete nano-electrospray source. Samples were loaded in Proxeon NanoES capillaries and ionized using IonSpray voltage of 900-1200 V. The instrument was calibrated using Glu-Fibrinopeptide B (Sigma-Aldrich) with fragment ions 246.15 and 1285.54 m/z. Peptide Mass Fingerprint (PMF) spectra were acquired in positive ion mode using a range of 450 - 1500 m/z. MS/MS data was obtained via the Information Dependent Acquisition (IDA) method where doubly and triply charged parent ions were selected for fragmentation by collision induced dissociation (CID), using nitrogen as collision gas. MS/MS data was searched against the msdb database (incorporating the sequence of flagellin, N-terminal FLAG and Sifuvirtide in the msdb.fasta file). Annotated MS/MS spectra are indicated for the identified peptides of interest with mass labels, y-ions and b-ions identified (Fig. 9A, B).The MS/MS spectra confirmed, with extremely high confidence, the presence of two overlapping peptides (1772.8372 Da, ILEESQEQQDRNER; Fig. 9A and 2243.0567 Da, ILEESQEQQDRNERDLLE; Fig. 9B) corresponding to the C-terminal region of Sifuvirtide, providing evidence for the successful expression of Sifuvirtide as an N-terminal peptide fusion.

### Application of host strain: Peptides secreted according to the invention as C-terminal flagellin fusions.

### EXAMPLE 5:

### 5.1 Expression as FliC C-terminal fusion of the anti-viral peptide, Sifuvirtide.

### 5.1.1 Construction of pSECNCFSif carrying the FLAG::Sifuvirtide peptide.

The plasmid pSECFliC containing the full FliC protein as well as its 5' and 3' regions as described by Crampton et al. (2007) was used as template for the primers σ^{D}Kpn and FliCendR to obtain a 1065 bp PCR product containing the full FliC protein with *Xho*I and *Bam*HI sites incorporated at the C-terminal end. This fragment (digested with *Xho*I and *Kpn*I) was ligated together with the FLAG-Sifuvirtide fragment (digested with *Xho*I and *Bam*HI) into pSECNF2Sif digested with *Kpn*I and *Bam*HI. The encoded polypeptide sequence of FliC with the antiviral protein, Sifuvirtide, fused to the C-terminal end, is provided as SEQ ID NO: 70. The construct encoding this polypeptide was named pSECNCFSif and is provided as SEQ ID NO: 71.The constructs were confirmed to be correct by PCR and sequencing analysis as described for pSECNFFuzC7 and then transformed into *B. halodurans* BhFD05.

### 5.1.2 Evaluation of the expression of the C-terminal peptide fusion.

The chimeric flagellin gene product of pSECNCFSif was evaluated for secretion into the extracellular medium of *B. halodurans* BhFD05 according to section 3.3.3.

The extracellular protein fraction was analysed on a 10% SDS-PAGE gel. There was a detectable chimeric flagellin protein band for the flagellin::Sifuvirtide fusion at the size range of ~37 kDa. The anti-viral peptide FLAG-Sifuvirtide, was extracted and purified from *B. halodurans* BhFD05 culture supernatant as described in section 3.3.4. Samples were run on a 10% SDS-PAGE gel (Fig 10).

### 5.1.3 Analysis of the C-terminal peptide fusion.

The purified immunogenic chimeric flagellin fusion protein was quantified using a Qubit^{™} fluorometer (Invitrogen) with Quant-iT^{™} protein assay reagents as described above. The total protein quantity obtained ranged between 5 and 12 mg immunogenic chimeric flagellin fusion protein per litre of culture supernatant, at an estimated 95% purity. The anti-viral chimeric flagellin fusion protein was electrophoresed on a 10% SDS-PAGE gel and bands were excised from the gel and subjected to enzymatic cleavage by modified sequencing grade trypsin through a method well known by those of skill in the art. The Sifuvirtide peptide presence and integrity were verified by mass-spectrometry analysis using a QSTAR^{®} Elite mass spectrometer (Applied Biosystems/MDS SCIEX) as described in section 4.1.3. Annotated MS/MS spectra are indicated for the identified peptides of interest with mass labels, y-ions and b-ions identified (Fig. 11A, B). The MS/MS spectra confirmed, with extremely high confidence, the presence of peptides corresponding to the N-terminal region of the anti-viral peptide LEESGADYKDDDDKGGVDMSWETWEREIENYTR (3938.6768 Da); Fig. 11A as well as the C-terminal region, ILEESQEQQDRNERDLLE (2243.0669 Da); Fig. 11B). These results provide evidence for the successful expression of Sifuvirtide as a C-terminal peptide fusion.

### REFERENCES

**Bolognesi DP, Matthews TJ and Wild CT (1995)**. Synthetic peptide inhibitors of HIV transmission. United States Patent 5,464,933
Crampton M, Berger E, Reid S and Louw M (2007). The development of a flagellin surface display expression system in a moderate thermophile, Bacillus halodurans Alk36. Appl. Microbiol. Biotechnol. 75: 559-607.
Franquelim HG, Loura LMS, Santos NC and Castanho MARB (2008). Sifuvirtide screens rigid membrane surfaces. Establishment of a correlation between efficacy and membrane domain selectivity among HIV fusion inhibitor peptides J. Am. Chem. Soc. 130: 6215-6223
Hancock REW and Leherer R (1998). Cationic peptides: a new source of antibiotics. TIBTECH 16: 82-88
Hewer R and Meyer D (2003). Peptide immunogens based on the envelope region of HIV-1 are recognized by HIV/AIDS patient polyclonal antibodies and induce strong humoral immune responses in mice and rabbits. Mol Immun 40: 327-335
Selsted ME, Novotny MJ, Morris WL, Tang Y-Q, Smith W and Cullor JS (1992). Indolicidin, a novel bacteriocidal tridecapeptide amide from neutrophils. J Biol Chem 267, 4292-4295
Shevchenko, A and Shevchenko, A (2001) Evaluation of the efficiency of in-gel digestion of proteins by peptide isotopic labeling and MALDI mass spectrometry, Anal Biochem 296: 279-283
Tang H-Y and Speicher DW (2004). Identification of alternative products and optimization of 2-nitro-5-thiocyanatobenzoic acid cyanylation and cleavage at cysteine residues. Anal Biochem 334: 48-61.

## Claims

1. A method of producing a flagellin-based chimeric protein, the method including
culturing a *B. halodurans* BhFD05 (*Δhag*, *ΔfliD*, *ΔwprA*, *Δalp*, *Δapr*, *Δvpr*, Δa*sp*) strain deposited under Accession Number 41533 at the NCIMB, and
causing the strain to express and secrete, into an extracellular growth medium, high levels of a flagellin-based chimeric protein comprising a heterologous peptide selected from Indolicidin, Enfuvirtide and Sifuvirtide, and (i) inserted in-frame into a flagellin variable region which is flanked on its N-terminal side by an N-terminal fragment of a flagellin polypeptide and, optionally, flanked on its C-terminal side by a C-terminal fragment of a flagellin polypeptide, or (ii) fused to the C-terminal of a flagellin polypeptide.

2. A method according to Claim 1, wherein the growth medium containing the chimeric protein, is usable as a crude preparation, with the chimeric protein being partially or fully purified from the growth medium.

3. A flagellin-based chimeric protein obtainable by the method of Claim 1 or Claim 2, and which comprises a heterologous peptide selected from Indolicidin, Enfuvirtide and Sifuvirtide, and (i) inserted in-frame into a flagellin variable region which is flanked on its N-terminal side by an N-terminal fragment of a flagellin polypeptide and, optionally, flanked on its C-terminal side by a C-terminal fragment of a flagellin polypeptide, or (ii) fused to the C-terminal of a flagellin polypeptide.

4. A flagellin-based chimeric protein according to Claim 3, wherein the heterologous peptide is fused only to the N-terminal fragment of a flagellin polypeptide.

5. A flagellin-based chimeric protein according to Claim 3, wherein the heterologous peptide is fused to the C-terminal of a flagellin polypeptide.

6. A flagellin-based chimeric protein according to any one of Claims 3 to 5 inclusive, which includes a polypeptide tag fused to the N-terminal side of the heterologous peptide.

7. A flagellin-based chimeric protein according to any one of Claims 3 to 6 inclusive, which includes at least one cleavage site linked to the heterologous peptide region.

8. Use of a flagellin-based chimeric protein according to any one of Claims 3 to 7 inclusive, in the manufacture of a medicament for therapeutic use.

9. A nucleic acid encoding a chimeric protein according to any one of Claims 3 to 7 inclusive, the nucleic acid comprising a nucleotide sequence encoding (i) the N-terminal fragment of a flagellin polypeptide; the variable region of a flagellin polypeptide; optionally, the C-terminal fragment of a flagellin polypeptide, and a nucleotide sequence encoding a heterologous polypeptide selected from Indolicidin, Enfuvirtide and Sifuvirtide, and inserted in-frame into the nucleotide sequence encoding the variable region of the flagellin polypeptide, or (ii) a heterologous polypeptide selected from Indolicidin, Enfuvirtide and Sifuvirtide, and fused to the C-terminal of a flagellin polypeptide.

10. A nucleic acid according to Claim 9, wherein the nucleotide sequence encoding the heterologous peptide is inserted immediately after any nucleotide between nucleotide 162 and nucleotide 606 of SEQ ID NO: 7.

11. An expression cassette which includes a nucleic acid sequence encoding the chimeric protein according to any one of Claims 3 to 7 inclusive.

12. An expression cassette according to Claim 11, wherein the nucleic acid sequence encoding the chimeric protein is integrated into the chromosome of the host cell.

13. A nucleic acid vector which includes a nucleic acid sequence encoding the chimeric protein of any one of Claims 3 to 7 inclusive, operably linked to a transcriptional regulatory element.

14. A nucleic acid vector according to Claim 13, which is an extra-chromosomal plasmid.

15. A bacterial cell containing the nucleic acid vector of Claim 13 or Claim 14.

16. A bacterial cell according to Claim 15, which is of the strain *B. halodurans* Alk36 (*Δhag*, *ΔfliD*, *ΔwprA*, *Δalp*, *Δapr*, *Δvpr*, *Δasp*) designated BhFD05 deposited under Accession Number 41533 at the NCIMB.

## Patentansprüche

1. Verfahren zur Herstellung eines auf Flagellin basierenden chimären Proteins, wobei das Verfahren umfasst
Kultivieren eines *B. halodurans* BhFD05 (*Δhag*, *ΔfliD*, *ΔwprA*, *Δalp*, *Δapr*, *Δvpr*, *Δasp*) Stamms, hinterlegt unter der Zugangsnummer 41533 beim NCIMB und
Bewirken, dass der Stamm große Mengen eines auf Flagellin basierenden chimären Proteins, umfassend ein heterologes Peptid, ausgewählt aus Indolicidin, Enfuvirtid und Sifuvirtid, und (i) "in-frame" eingefügt in eine variable Region eines Flagellins, welche auf ihrer N-terminalen Seite an ein N-terminales Fragment eines Flagellinpolypeptids angrenzt und welche gegebenenfalls auf ihrer C-terminalen Seite an ein C-terminales Fragment eines Flagellinpolypeptids angrenzt oder (ii) an den C-Terminus des Flagellinpolypeptids fusioniert ist, exprimiert und in ein extrazelluläres Wachstumsmedium sekretiert.

2. Verfahren nach Anspruch 1, worin das das chimäre Protein enthaltende Wachstumsmedium als grobe Holzzubereitung verwendbar ist und das chimäre Protein teilweise oder vollständig aus dem Wachstumsmedium gereinigt werden kann.

3. Auf Flagellin basierendes chimäres Protein, erhältlich durch ein Verfahren nach Anspruch 1 oder Anspruch 2, und das ein heterologes Peptid, ausgewählt aus Indolicidin, Enfuvirtid und Sifuvirtid, (i) "in-frame" eingefügt in eine variable Region eines Flagellins, welche auf ihrer N-terminalen Seite an ein N-terminales Fragment eines Flagellinpolypeptids angrenzt und welche gegebenenfalls auf ihrer C-terminalen Seite an ein C-terminales Fragment eines Flagellinpolypeptids angrenzt oder (ii) an den C-Terminus des Flagellinpolypeptids fusioniert ist, enthält.

4. Auf Flagellin basierendes chimäres Protein nach Anspruch 3, worin das heterologe Peptid nur an das N-terminale Fragment eines Flagellinpolypeptids fusioniert ist.

5. Auf Flagellin basierendes chimäres Protein nach Anspruch 3, worin das heterologe Peptid an den C-Terminus eines Flagellinpolypeptids fusioniert ist.

6. Auf Flagellin basierendes chimäres Protein nach einem der Ansprüche 3 bis einschließlich 5, das einen an die N-terminale Seite des heterologen Peptids fusionierten Polypeptid-Tag enthält.

7. Auf Flagellin basierendes chimäres Protein nach einem der Ansprüche 3 bis einschließlich 6, das mindestens eine mit der heterologen Peptidregion verbundene Spaltstelle enthält.

8. Verwendung eines auf Flagellin basierenden chimären Proteins nach einem der Ansprüche 3 bis einschließlich 7 zur Herstellung eines Medikaments für die therapeutische Verwendung.

9. Nukleinsäure, die das chimäre Protein nach einem der Ansprüche 3 bis einschließlich 7 kodiert, wobei die Nukleinsäure eine Nukleotidsequenz umfasst, die (i) das N-terminale Fragment eines Flagellinpolypeptids, die variable Region eines Flagellinpolypeptids, gegebenenfalls das C-terminale Fragment eines Flagellinpolypeptids kodiert und eine Nukleotidsequenz, die ein heterologes Polypeptid, ausgewählt aus Indolicidin, Enfuvirtid und Sifuvirtid, kodiert und "in-frame" in die Nukleotidsequenz, die die variable Region des Flagellinpolypeptids kodiert, eingefügt ist oder (ii) ein heterologes Polypeptid kodiert, ausgewählt aus Indolicidin, Enfuvirtid and Sifuvirtid, und mit dem C-Terminus eines Flagellinpolypeptids verbunden ist.

10. Nukleinsäure nach Anspruch 9, wobei die das heterologe Peptid kodierende Nukleotidsequenz direkt hinter einem der Nukleotide zwischen Nukleotid 162 und Nukleotid 606 der SEQ ID NO: 7 eingefügt ist.

11. Expressionkassette, die eine das chimäre Protein nach einem der Ansprüche 3 bis einschließlich 7 kodierende Nukleinsäuresequenz enthält.

12. Expressionkassette, nach Anspruch 11, wobei die das chimäre Protein nach einem der Ansprüche 3 bis einschließlich 7 kodierende Nukleinsäuresequenz in das Chromosom der Wirtszelle integriert wird.

13. Nukleinsäurevektor, der eine das chimäre Protein nach einem der Ansprüche 3 bis einschließlich 7 kodierende Nukleinsäure enthält, die in operativer Weise mit einem Transkriptionsregulationselement verbunden ist.

14. Nukleinsäurevektor nach Anspruch 13, der ein extrachromosomales Plasmid ist.

15. Bakterienzelle, umfassend den Nukleinsäurevektor der Ansprüche 13 oder 14.

16. Bakterienzelle nach Anspruch 15, die zu dem mit BhFD05 bezeichneten und unter der Zugangsnummer 41533 beim NCIMB hinterlegten *B. halodurans* Alk36 (Δ*hag,* Δ*fliD,* ΔwprA, Δ*alp,* Δ*apr,* Δ*vpr, Δasp*) Stamm gehört.

## Revendications

1. Procédé de production d'une protéine chimérique à base de flagelline, lequel procédé comporte les étapes suivantes :
- cultiver une souche *B. halodurans* BhFD05 (Δ*hag,* Δ*fliD, ΔwprA,* Δ*alp,* Δ*apr, Δvpr,* Δ*asp*), déposée auprès de la NCIMB avec le numéro de référence 41533 ;
- et faire en sorte que cette souche exprime et sécrète, dans le milieu de croissance extracellulaire, de grandes quantités d'une protéine chimérique à base de flagelline, comprenant un peptide hétérologue choisi parmi l'indolicidine, l'enfuvirtide et le sifuvirtide, et
i) inséré, dans le cadre de lecture, dans une région variable de flagelline qui est flanquée du côté N-terminal par un fragment N-terminal d'un polypeptide flagelline, et en option, flanquée du côté C-terminal par un fragment C-terminal d'un polypeptide flagelline,
ii) ou fusionné à l'extrémité C-terminale d'un polypeptide flagelline.

2. Procédé conforme à la revendication 1, dans lequel le milieu de croissance contenant la protéine chimérique est utilisable en tant que préparation brute, la protéine chimérique étant en partie ou en totalité retirée du milieu de croissance lors de sa purification.

3. Protéine chimérique à base de flagelline, accessible par un procédé conforme à la revendication 1 ou 2, et comprenant un peptide hétérologue choisi parmi l'indolicidine, l'enfuvirtide et le sifuvirtide, qui est
i) inséré, dans le cadre de lecture, dans une région variable de flagelline qui est flanquée du côté N-terminal par un fragment N-terminal d'un polypeptide flagelline, et en option, flanquée du côté C-terminal par un fragment C-terminal d'un polypeptide flagelline,
ii) ou fusionné à l'extrémité C-terminale d'un polypeptide flagelline.

4. Protéine chimérique à base de flagelline, conforme à la revendication 3, dans laquelle le peptide hétérologue est fusionné seulement au fragment N-terminal d'un polypeptide flagelline.

5. Protéine chimérique à base de flagelline, conforme à la revendication 3, dans laquelle le peptide hétérologue est fusionné à l'extrémité C-terminale d'un polypeptide flagelline.

6. Protéine chimérique à base de flagelline, conforme à l'une des revendications 3 à 5, bornes comprises, qui comprend un polypeptide étiquette fusionné au côté N-terminal du peptide hétérologue.

7. Protéine chimérique à base de flagelline, conforme à l'une des revendications 3 à 6, bornes comprises, qui comprend au moins un site de clivage raccordé à la région du peptide hétérologue.

8. Utilisation d'une protéine chimérique à base de flagelline, conforme à l'une des revendications 3 à 7, bornes comprises, dans la fabrication d'un médicament à usage thérapeutique.

9. Acide nucléique codant une protéine chimérique conforme à l'une des revendications 3 à 7, bornes comprises, lequel acide nucléique comprend une séquence de nucléotides codant :
i) le fragment N-terminal d'un polypeptide flagelline, la région variable d'un polypeptide flagelline, et en option, le fragment C-terminal d'un polypeptide flagelline,
ainsi qu'une séquence de nucléotides codant un peptide hétérologue choisi parmi l'indolicidine, l'enfuvirtide et le sifuvirtide et insérée, dans le cadre de lecture, dans la séquence de nucléotides codant la région variable du polypeptide flagelline
ii) ou un peptide hétérologue choisi parmi l'indolicidine, l'enfuvirtide et le sifuvirtide, fusionné à l'extrémité C-terminale d'un polypeptide flagellin.

10. Acide nucléique conforme à la revendication 9, dans lequel la séquence de nucléotides codant le peptide hétérologue est insérée immédiatement après n'importe quel nucléotide situé entre le nucléotide 162 et le nucléotide 606 de la Séquence N° 7.

11. Cassette d'expression qui renferme une séquence d'acide nucléique codant une protéine chimérique conforme à l'une des revendications 3 à 7, bornes comprises.

12. Cassette d'expression conforme à la revendication 11, par laquelle la séquence d'acide nucléique codant la protéine chimérique est intégrée dans le chromosome de la cellule hôte.

13. Vecteur d'acide nucléique qui contient une séquence d'acide nucléique codant une protéine chimérique conforme à l'une des revendications 3 à 7, bornes comprises, opérationnellement raccordée à un élément de régulation de la transcription.

14. Vecteur d'acide nucléique conforme à la revendication 13, qui est un plasmide extra-chromosomique.

15. Cellule bactérienne contenant un vecteur d'acide nucléique conforme à la revendication 13 ou 14.

16. Cellule bactérienne conforme à la revendication 15, qui est une cellule de la souche *B. halodurans* BhFD05 (Δ*hag,* Δ*fliD,* Δ*wprA,* Δ*alp,* Δ*apr,* Δ*vpr,* Δ*asp*), déposée auprès de la NCIMB avec le numéro de référence 41533.
